# EUROPEAN PATENT APPLICATION

(11) **EP 2 799 056 A1**
(43) Date of publication of application: **05.11.2014**
(21) Application number: 12862650.4
(22) Date of filing: 26.12.2012
(51) Int. Cl.: A61J 1/10, A61J 3/00, B65D 33/14, B65D 33/17, B65D 33/25, B65D 33/36, B65D 65/04, B67B 7/48

(54) **EXPOSURE PREVENTION COVER, EXPOSURE PREVENTION COVER MODULE PROVIDED WITH SAME, DRUG SOLUTION SUPPLY SYSTEM, AND DRUG SOLUTION SUPPLY METHOD**

(30) Priority: 29.12.2011 JP 2011290435; 22.03.2012 JP 2012065914
(71) Applicant: Otsuka Pharmaceutical Factory, Inc., Naruto-shi, Tokushima 772-8601 (JP); Otsuka Techno Corporation, Tokushima 771-0360 (JP)
(72) Inventor: INOUE, Fujio, Naruto-shi Tokushima 772-8601 (JP); SHIGA, Junya, Naruto-shi Tokushima 772-8601 (JP); NISHIOKA, Masaki, Naka-gun Tokushima 771-5209 (JP); TAKIGUCHI, Osamu, Naka-gun Tokushima 771-5209 (JP); HAMAI, Katsuyoshi, Naka-gun Tokushima 771-5209 (JP)
(74) Representative: Webster, Jeremy Mark
(86) International application number: PCT/JP2012/083654
(87) International publication number: WO 2013/099946

(57) **Abstract**

The present invention provides an exposure prevention cover that prevents exposure to a drug solution, pharmaceuticals or the like, an exposure prevention cover module provided with the same, a drug solution supply system, and a drug solution supply method. An exposure prevention cover for housing a drug solution container that contains a drug solution and has a connection port through which the drug solution can be discharged, includes a cover body that houses the drug solution container and has an opening open to the outside for inserting the drug solution container; a closure section that closes the opening; a holding section that holds the drug solution container housed in the cover body inside the cover body; a connection section that is attached to the cover body and can be connected to the discharge port of the drug solution container, so that the drug solution in the drug solution container can be discharged outside the cover body; and a suspension section provided on the cover body for suspending the cover body.

## Description

### [Technical Field]

The present invention relates to an exposure prevention cover that prevents exposure to a drug solution, pharmaceuticals or the like, an exposure prevention cover module provided with the same, a drug solution supply system, and a drug solution supply method.

### [Background Art]

Among drug solutions administered to patients, there are some that have a strong effect. For example, while an anticancer drug has an anticancer effect, it contains a toxic component that may even cause damage to normal cells. For this reason, during preparation work of a drug solution, close attention is paid to the prevention of the drug solution coming into contact with to a human body. During administration of an anticancer drug to a patient, also, close attention is required as to the splashing of droplets on the medical worker and the patient. However, it is difficult to avoid the splashing of fine droplets, and there is apprehension that a medical worker who has been continuously exposed to a minute amount of droplets over a long period of time may impair his or her health.

There have been no such things as external containers and external bags for preventing exposure to splashing and falling of such droplets. Attention has conventionally been paid to the mixing of outside air into a drug solution causing alteration during storage of the drug solution's container. For example, an example of an external bag made of a plastic sheet in which a drug solution container is enclosed is shown in FIG. 21 (Patent Literature 1). In an external bag 901 made of an oxygen-barrier plastic sheet, drug solution containers 903A and 903B are enclosed. During preparation processing (mixing of a vitamin, etc.), a needle of a syringe 906 is inserted into the bag via a puncture cap 902 formed integrally with a rubber body. The external bag 901 prohibits outside air from entering inside, thereby preventing the drug solution from altering due to the mixing of oxygen in the external bag 901 during storage of the drug solution containers 903A and 903B.

However, use of such an external bag does not prevent exposure to splashed droplets. During administration of the drug solution to a patient, the external bag 901 is cut open along a line between notches 914 at the right end portion, and the drug solution container is taken out from the external bag 901 and used. When the drug solution container is connected to a drug solution line to the patient in this state, leakage of the drug solution from the connection may occur. Also, even if the drug solution is administered via the puncture cap 902 during administration of the drug solution to the patient, leakage and splashing of the drug solution may occur at the time of insertion of the needle into the puncture cap 902 and at the time when the needle is inserted or removed, such as termination of the administration and replacement of the drug solution container, causing the possibility of exposure to a human body. Moreover, sufficient consideration must be taken to the disposal of a used needle to which an anticancer drug, etc. has become attached, as with a needle-stick accident and the handling of such a needle as medical waste. Such a problem can occur, not only in the drug solution described above, but also in drug solutions and drugs in general that will affect the user when the user is exposed thereto.

### [Citation List]

### [Patent Literature]

[PTL 1] Japanese Patent No. 4481563

### [Summary of Invention]

### [Technical Problem]

In view of the problem described above, an objective of the present invention is to provide an exposure prevention cover that prevents exposure to a drug solution, pharmaceuticals, etc., an exposure prevention cover module provided with the same, a drug solution supply system, and a drug solution supply method.

### [Means for Solving the Problem and Effects of the Invention]

In order to achieve the above objective, the first exposure prevention cover according to the present invention is an exposure prevention cover including: a holding section that holds a drug solution container; a cover body 1, 31 for enclosing the drug solution container by surrounding the container; a suspension section that suspends the cover body 1, 31; and a connection section that can be connected to a connection port of the drug solution container. The cover body 1, 31 has an opening 1B, 31B provided on one face, and a closure section that closes the opening 1B, 31B is provided. With this configuration, by closing the cover body after insertion of the drug solution container in the cover body, it is possible to enclose the splashing of droplets inside the cover body and also dispose of the drug solution container while being kept enclosed in the cover body.

According to the second exposure prevention cover of the present invention, the cover body 1, 31 can be made of a material having at least one of a light transmittance property, a gas barrier property, a waterproof property, and a light shielding property. With this configuration, the visibility of the drug solution container suspended in the cover body is secured, so that the work of connecting the connection port of the drug solution container with the connection section can be easily performed from outside the cover body. Also, any external effect of oxygen, etc. on the drug solution can be prevented. In addition, the passage of evaporated drug solution or the drug solution itself from inside is prevented thereby deterring splashing of the drug solution, and thus exposure of the drug solution to the user can be avoided.

According to the third exposure prevention cover of the present invention, the closure section can be comprised of a clip that fastens the opening 1B, 31B from both sides. With this configuration, the opening can be easily closed with the clip.

According to the fourth exposure prevention cover of the present invention, the closure section can be comprised of a zipper seal capable of closing the opening 1B, 31B. With this configuration, the opening can be closed without the use of another member.

According to the fifth exposure prevention cover of the present invention, at least part of the cover body 1, 31 can have a combination of materials having a water absorption property. With this configuration, a pool of the drug solution having leaked from the drug solution container can be absorbed.

According to the sixth exposure prevention cover of the present invention, at least one suspension section that suspends the cover body 1, 31 with the drug solution container housed therein can be provided on an upper part of the cover body 1, 31. With this configuration, the exposure prevention cover can be stabilized when being suspended on a drip stand.

According to the seventh exposure prevention cover of the present invention, the cover body 31 can have a volume capable of housing only one drug solution container. With this configuration, when one drug solution container is used, the cover body 31 having a volume capable of housing one drug solution container can be provided. Thus, such cover bodies can be used for a number of drug solution containers.

According to the eighth exposure prevention cover of the present invention, the holding section can be a lower holding section 32 having an opening size that allows insertion of the connection port protruding from the drug solution container housed in the cover body 31 but does not allow insertion of a shoulder of the drug solution container near the connection port. With this configuration, the drug solution container can be stably held in the cover body by being supported from below.

According to the ninth exposure prevention cover of the present invention, the holding section can be a container holder 2 that engages with a hanging hole formed on the drug solution container in the cover body 1, 31 to be able to suspend the drug solution container. With this configuration, a plurality of drug solution containers can be held by being suspended with their hanging holes.

According to the tenth exposure prevention cover of the present invention, the container holder 2 can have an upper holding section 2D that is inserted through the hanging hole formed on the drug solution container so as to be able to suspend the drug solution container, and the top surface of the upper holding section 2D can be formed so as to be uneven. With this configuration, the drug solution container can be held in its suspended state by the container holder, and can be avoided from displacing in the exposure prevention cover.

According to the eleventh exposure prevention cover of the present invention, the connection section further can include a needle body 3, 33 for being inserted into the connection port of the drug solution container, and a needle cover 4, 34 that has a cylindrical shape surrounding the needle body 3, 33 and extends longer than the height of the needle body 3, 33. With this configuration, by surrounding the needle body with the needle cover, the tip of the needle body is prevented from being exposed, and thus an occurrence that the exposure prevention cover may be damaged with the needle body can be avoided.

According to the twelfth exposure prevention cover of the present invention, the needle body 3, 33 can be provided with an anti-falling structure for preventing the needle body 3, 33 from falling after being inserted into the connection port of the drug solution container. With this configuration, the needle body inserted into the connection port of the drug solution container can be prevented from falling due to inadvertent pulling of a drug solution administration line, etc.

According to the thirteenth exposure prevention cover of the present invention, the needle cover 4, 34 can be made of a flexible material. With this configuration, in puncturing the connection port of the drug solution container with the needle body, even when the connection port of the drug solution container fails to fit in the inner diameter of the needle cover, a puncture can be made by pressing the needle cover like crushing it.

According to the fourteenth exposure prevention cover of the present invention, a simple partition 38 that seals the needle body 3, 33 and the needle cover 4, 34 in the cover body 1, 31 can be provided. With this configuration, until the simple partition 38 is opened to use the exposure prevention cover, the needle tip and the needle cover can avoid contact with the outside air and thus be kept in an aseptic state.

The exposure prevention cover module according to the present invention includes: any of the exposure prevention covers described above; a backflow restriction member that is attached to the connection section of the exposure prevention cover to restrict backflow of a liquid; and a drug solution discharge line attached to the connection section via the backflow restriction member. The backflow restriction member is not specifically limited as long as it can prevent backflow of a liquid, but a check valve, a clip, a drip chamber, etc., for example, can be used.

At least one port via which a liquid can be injected can be provided in the drug solution discharge line. For example, a port for injection of a flushing liquid and a port for coupling a drug solution discharge line of another exposure prevention cover module can be provided. In particular, with a port for coupling, a plurality of drug solutions can be supplied through one line, and this can simplify the device.

The drug solution supply system according to the present invention includes: any of the exposure prevention covers described above; at least one drug solution container housed in the exposure prevention cover; an infusion container containing an infusion solution; an infusion solution discharge line connected to the infusion container at one end to discharge the infusion solution from the infusion container; a drug solution discharge line connected to the connection section of the exposure prevention cover at one end to discharge a drug solution in the drug solution container housed in the exposure prevention cover; a coupling section that couples the other end of the drug solution discharge line and the other end of the infusion solution discharge line; a supply line connected to the coupling section to discharge a liquid from at least one of the infusion solution discharge line and the drug solution discharge line; and a control member that controls flow of a liquid in at least one of the lines.

According to the system described above, by adjusting any of the control members, the infusion solution can be supplied from the infusion container to the drug solution discharge line before discharge of the drug solution. Therefore, any gas remaining in the drug solution discharge line can be expelled into the exposure prevention cover. That is, since the connection section and the drug solution container are not connected in the exposure prevention cover at this time, the gas is introduced into the exposure prevention cover. As the infusion solution, a basic solution such as a normal saline solution and a Ringer's solution can be used.

The above-described system can further include an infusion solution injection member attached to a portion of the drug solution discharge line near a junction with the connection section for injecting an infusion solution into the drug solution discharge line. With this configuration, since the drug solution remaining in the drug solution discharge line can be pushed out by injecting the infusion solution, all of the intended quantity of the drug solution can be supplied.

The above-described system can further include an air removal filter that is attached to the drug solution discharge line and allows passage of the drug solution flowing from the drug solution container but does not allow passage of air. With such an air removal filter provided, the work of supplying the infusion solution from the infusion container to the drug solution discharge line to expel gas remaining in the drug solution discharge line is unnecessary. Therefore, since the drug solution can be discharged without the gas expelling work, the work time can be shortened.

In the above-described system, the following configuration can be provided in place of the air removal filter. That is, the system can further include: an air exhaust filter that is attached to the drug solution discharge line and allows passage of the drug solution flowing from the drug solution container but exhausts air outside; and an air collection container that seals air exhausted from the air exhaust filter. By having this configuration, also, the work of expelling gas remaining in the drug solution discharge line is unnecessary. Also, since the air exhausted from the air exhaust filter may sometimes be altered by the drug solution, it is sealed inside the air collection container.

In the above-described system, an additional module for supplying a plurality of drug solutions can be further provided. The additional module includes at least one of the exposure prevention covers described above, a drug solution container housed in each of the at least one exposure prevention cover; and an additional drug solution discharge line connected, at one end, to the connection section of the at least one exposure prevention cover. One of the additional drug solution discharge lines can be connected to the drug solution discharge line as an additional discharge line. With this configuration, since the above drug solution discharge line and the additional drug solution discharge line of the additional module are serially connected, a plurality of drug solutions can be supplied through one route.

The additional module can have a plurality of exposure prevention covers, and the plurality of additional drug solution discharge lines can be serially connected to each other with the additional discharge line being at the head. With this configuration, a plurality of drug solution containers can be housed in the additional module, and, by serially connecting the lines, a plurality of drug solutions can be supplied through one route.

The drug solution supply method according to the present invention includes: a first preparation step of preparing at least one drug solution container containing a drug solution and having a connection port via which the drug solution can be discharged; a second preparation step of housing and sealing the drug solution container in an exposure prevention cover having a connection section via which the drug solution can be discharged; a third preparation step of connecting a drug solution line to the connection section; and a discharge step of connecting the connection section to the connection port of the drug solution container so as to discharge the drug solution in the drug solution container into the drug solution line via the connection section.

The drug solution supply method described above can further include: a fourth preparation step of preparing an infusion container containing an infusion solution; a fifth preparation step of connecting the infusion container to an infusion line that discharges the infusion solution from the infusion container; and a step of forming a supply line capable of discharging at least one of the infusion solution and the drug solution by connecting the drug solution line and the infusion line in a trifurcated fashion. The method can further include before the discharge step: a first priming step of discharging the infusion solution from the infusion container toward the supply line; and a second priming step of allowing the infusion solution to flow from the infusion container toward the connection section of the exposure prevention cover.

The above-described drug solution supply method can further include, after the discharge step, a flushing step of supplying an infusion solution at a position of the drug solution line near the connection section to allow the infusion solution to flow into the drug solution line.

In the above-described drug solution supply method, a plurality of drug solutions can be supplied. That is, a plurality of drug solution containers can be prepared in the first preparation step, each of the plurality of drug solution containers can be housed and sealed in the exposure prevention cover in the second preparation step, the drug solution line can be connected to the connection section of each of the exposure prevention covers and such drug solution lines can be serially connected in the third preparation step, and drug solutions can be sequentially discharged from the drug solution containers in the discharge step.

The above-described drug solution supply method can further include: a fourth preparation step of preparing an infusion container containing an infusion solution; a fifth preparation step of connecting the infusion container to an infusion line that discharges the infusion solution from the infusion container; and a step of forming a supply line capable of discharging at least one of the infusion solution and the drug solution by connecting the infusion line and the drug solution line placed at the head in a trifurcated fashion. A first priming step of discharging the infusion solution from the infusion container toward the supply line can be executed before the step of discharging the drug solution to the head drug solution line, and a second priming step of allowing the infusion solution to flow from the infusion container toward the connection section of the exposure prevention cover can be executed before the step of discharging the drug solution to each of the drug solution lines.

The above-described drug solution supply method can further include a flushing step of supplying an infusion solution at a position of each of the drug solution lines near the connection section to allow the infusion solution to flow into the drug solution line, after each discharge of the drug solution from the drug solution container in the discharge step.

In the above-described drug solution supply method, a plurality of drug solution containers can be housed and sealed in the exposure prevention cover in the second preparation step, and the discharge step can be executed for each of the drug solution containers. That is, a plurality of drug solution containers can be housed in one exposure prevention cover to perform supply of drug solutions.

In the above case, the above-described drug solution supply method can further include: a fourth preparation step of preparing an infusion container containing an infusion solution; a fifth preparation step of connecting the infusion container to an infusion line that discharges the infusion solution from the infusion container; and a step of forming a discharge section capable of discharging at least one of the infusion solution and the drug solution by connecting the drug solution line and the infusion line in a trifurcated fashion. A first priming step of discharging the infusion solution from the infusion container toward the discharge section can be executed before the step of discharging the drug solution to the head drug solution line, and a second priming step of allowing the infusion solution to flow from the infusion container toward the connection section of the exposure prevention cover can be executed before the step of discharging the drug solution from each of the drug solution containers.

The above-described drug solution supply method can further include a flushing step of supplying an infusion solution at a position of each of the drug solution lines near the connection section to allow the infusion solution to flow into the drug solution line, after each discharge of the drug solution from the drug solution containers in the discharge step.

In the above-described drug solution supply method, the following configuration can be used in place of executing the second priming step. That is, the method can further include: a fourth preparation step of preparing an infusion container containing an infusion solution; a fifth preparation step of connecting the infusion container to an infusion line that discharges the infusion solution from the infusion container; a step of forming a supply line capable of discharging at least one of the infusion solution and the drug solution by connecting the drug solution line and the infusion line in a trifurcated fashion; and a step of attaching, to the drug solution discharge line, a filter that allows passage of the drug solution flowing from the drug solution container but does not allow passage of air. The method can further include, before the discharge step, a first priming step of discharging the infusion solution from the infusion container toward the supply line.

According to the above method, the second priming step is unnecessary, and the drug solution can be discharged immediately after the first priming step. Therefore, the work time required for discharge of the drug solution can be shortened. Such a filter can also be applied to all of the drug solution supply methods described above in place of the second priming.

### [Brief Description of Drawings]

FIG. 1 is an external perspective view showing an exposure prevention cover according to the first embodiment of the present invention.
FIG. 2 is an enlarged perspective view showing a needle body as part of the exposure prevention cover.
FIGS. 3(a) and 3(b) are enlarged cross-sectional views respectively showing the state before and after connection of the needle body to a drug solution container.
FIG. 4 is an external perspective view showing the state where drug solution containers are suspended in the exposure prevention cover and an opening is closed with a clip.
FIG. 5 is an external perspective view showing the state where drug solution containers are suspended in the exposure prevention cover and an opening is closed with a zipper seal.
FIG. 6 is a schematic view showing a primary use example of the exposure prevention cover.
FIG. 7 is a schematic view showing a secondary use example of the exposure prevention cover.
FIG. 8 is an external perspective view showing a state where a water absorption sheet is provided in the bottom of the exposure prevention cover as an example.
FIG. 9 is an external front view showing an exposure prevention cover according to the second embodiment of the present invention.
FIG. 10 is an external front view showing the state where drug solution containers are inserted in the exposure prevention cover in FIG. 9.
FIG. 11 is an external front view showing a variation of the exposure prevention cover in FIG. 9.
FIG. 12 is a front view of a cover module according to the third embodiment of the present invention.
FIG. 13 is a front view of an infusion container and its accessories for supplying a normal saline solution.
FIG. 14 is a view showing a drug solution supply method according to the third embodiment.
FIG. 15 is a view showing the drug solution supply method according to the third embodiment.
FIG. 16 is a view showing the drug solution supply method according to the third embodiment.
FIG. 17 is a view showing the drug solution supply method according to the third embodiment.
FIG. 18 is a view showing the drug solution supply method according to the third embodiment.
FIG. 19 is a view showing another drug solution supply method according to the third embodiment.
FIG. 20 is a view showing yet another drug solution supply method according to the third embodiment.

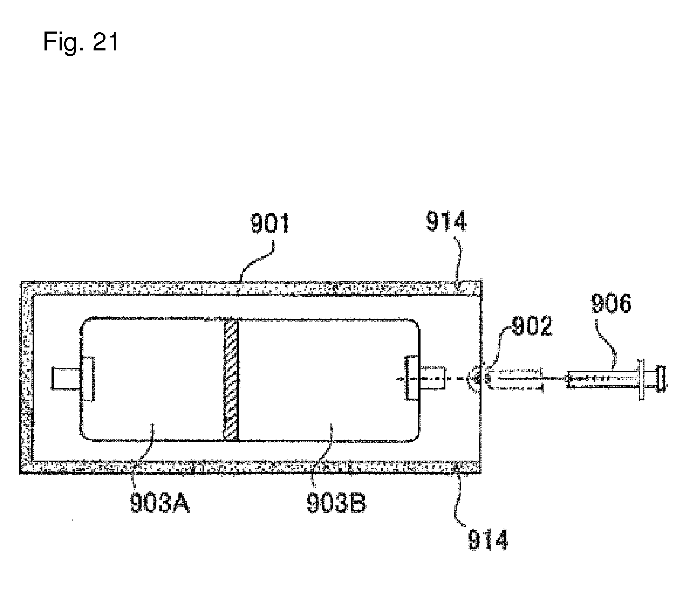

FIG. 21 is a cross-sectional view of a conventional drug solution container package.

### [Description of Embodiments]

Embodiments of the present invention will be described hereinafter with reference to the accompanying drawings. Note that the embodiments to be described hereinafter merely illustrate the exposure prevention cover for embodying the technical ideas according to the present invention, and are not intended to limit the exposure prevention cover according to the present invention. The members defined in the claims should never be limited to the members in the embodiments. In particular, unless otherwise specified, the sizes, materials, shapes, and relative placements of the composing members described in the embodiments are not intended to limit the scope of the invention, but mere illustrative examples. Note also that the sizes and positional relations of the members shown in the drawings are sometimes exaggerated to clarify the description. Further, in the following description, the same names and characters denote the same members or members of the same nature, and thus detailed description of such members are omitted appropriately. Moreover, as for the components constituting the present invention, a plurality of components may be formed of one member, so that the member serves as the plurality of components. In reverse, the function of one member may be borne by a plurality of members and achieved. Note also that the details described in one example or embodiment sometimes apply to other examples or embodiments.

### <1. First Embodiment>

### (1-1 Configuration of Exposure Prevention Cover)

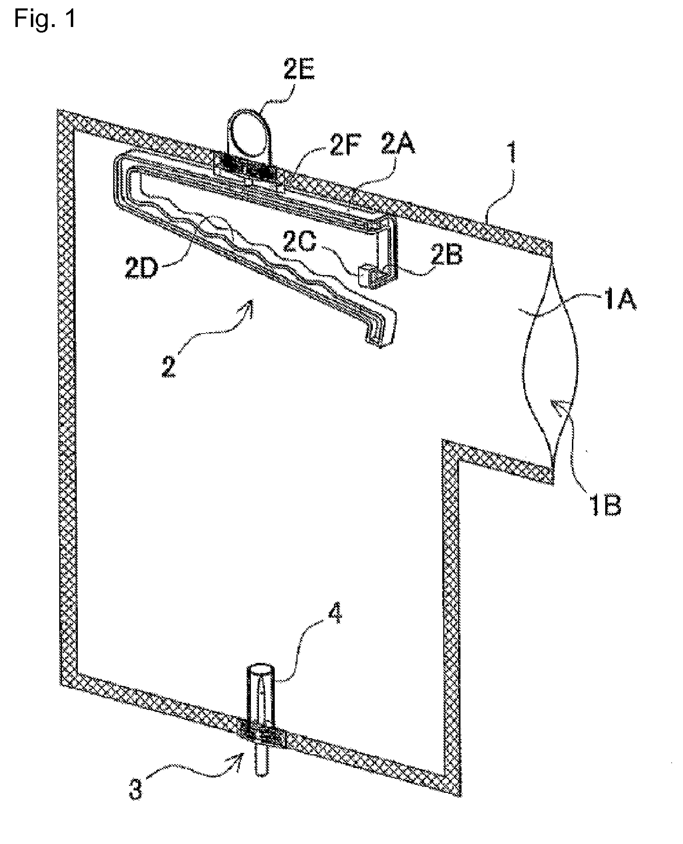

The configuration of an exposure prevention cover according to the first embodiment in which a container filled with a drug solution such as an anticancer drug is enclosed will be described with reference to the external perspective view of FIG. 1. First, the exposure prevention cover includes a cover body 1 that surrounds a drug solution container so as to enclose it. The exposure prevention cover also includes a hanger-shaped container holder 2 that can suspend the drug solution container as a holding section and a suspension section, a needle body 3 that can be inserted into a connection port of the drug solution container, and a needle cover 4 that has a cylindrical shape surrounding the needle body 3 and extends longer than the height of the needle body 3. The cover body 1 has an opening 1B through which the drug solution container can be inserted. The drug solution container is inserted through the opening 1B and suspended by the container holder 2, and thereafter the cover body 1 is closed with a closure member (closure section) that closes the opening 1B.

### (1-2 Method of Producing Exposure Prevention Cover)

The cover body 1 is integrally formed by thermally welding the periphery thereof except for the opening 1B with part of the container holder 2 and the needle body 3 being inserted between sheets of the cover body 1. The welding of the cover body is not limited to thermal welding, but adhesives, etc. can also be used. In FIG. 1, the thermally welded portion is represented by crosshatching for easy recognition. Although not shown, the welded portion may be formed in a portion slightly away from the periphery of the cover body, not in the periphery, leaving the periphery of the cover body being unbonded. This prevents the edge of the cover body from becoming rigid by welding, and thus can avoid an occurrence that a hand or the like of the medical worker may be hit by a corner edge of the cover body.

### (1-3 Cover Body 1)

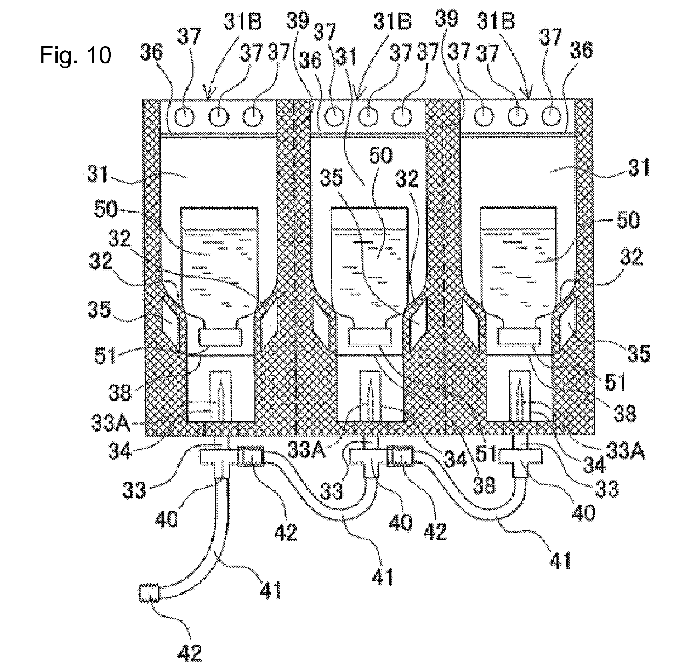

The cover body 1 according to this embodiment has an approximately rectangular shape as a whole with a protrusion 1A formed at the upper right position, as shown in FIG. 1. The protrusion 1A is provided for closing the opening 1B through which the drug solution container is inserted with a closure member to be described later. Providing the opening 1B at the edge of the protrusion 1A as described above makes it easy to close only part of the cover body 1, and not the entire end edge of the cover body. The periphery of the cover body except for the opening is thermally welded as described above. The shape of the cover body is not limited to that described above, but can be shaped to accommodate the drug solution container to be housed. For example, it may have a shape of a square, an ellipse, a trapezoid, etc. The cover body 1 can also have an easily openable simple partition above the needle tip and the needle cover, as shown in FIG. 10. The simple partition is formed as an easy peel-off seal by thermally welding the inner faces of the cover together. With this partition, it is possible to prevent the needle tip and the needle cover from coming into contact with the outside air until the exposure prevention cover is used, keeping them in an aseptic condition.

It is preferable that the material of the exposure prevention cover body 1 has at least one function of a light transmittance property, a gas barrier property, a waterproof property, and a light shielding property. As the material having the light transmittance property, polypropylene (PP), polyethylene (PE), etc. can be used. With the light transmittance property, the work of connecting the connection port of the drug solution container with the needle body 3 in the cover body 1 to be described later can be performed easily even from outside the cover body. Note that, in order to avoid degradation of the quality of the drug solution, the cover body 1 can be made of a material having a wavelength-selective light shielding property and a partial or entire light shielding property, to avoid transmission of light having a specific wavelength. Examples of such a material include an aluminum laminated film, a material with an UV absorbent mixed or applied, and a material having a light-shading ink layer. As a material having the gas barrier property, polyethylene terephthalate (PET) and polyamide on which an inorganic substance is evaporated, aluminum foil, etc. can be used. In particular, by using aluminum oxide or silicon as the metal for evaporation, the light transmittance property can also be combined. With the gas barrier property, it is possible to prevent evaporated drug solution and the drug solution itself from leaking outside the cover body 1. Also, as a material having the waterproof property, polyamide (PA), etc. can be used in addition to the above materials. With the waterproof property, it is possible to avoid exposure of a drug having strong effects, such as an anticancer drug, to the user. Silicon oxide may be mixed in the material constituting the inside of the cover body so as to have a good sliding property so that the drug solution container can be easily inserted into the cover body. Note that it is preferable to use a material that can be subjected to steam sterilization, gamma ray sterilization, and electron beam sterilization as the material of the cover body 1.

### (1-4 Container Holder 2)

The exposure prevention cover includes the container holder 2 as the holding section for suspending the drug solution container. The container holder 2 also serves as the suspension section that suspends the exposure prevention cover from a drip stand, etc. The container holder 2 shown in FIG. 1 has a hanger-like shape configured to be able to suspend a drug solution container inside the cover body 1. The exposure prevention cover includes a support 2A having a hanger-like width inside the cover. The support 2A has an upper holding section 2D curved into a U-shape at its left as shown in FIG. 1. The support 2A also has a latch section 2B that is bent downward at a right angle and further protrudes frontward by a given length at a right angle to be perpendicular to the support 2A, as shown on the right of FIG. 1. The upper holding section 2D is formed to be slightly longer than the width of the upper portion of the support 2A, so that the right end of the upper holding section 2D can be latched on the latch section 2B. The upper holding section 2D functions as the holding section that holds a drug solution container. Further, a protrusion piece 2C is formed to protrude upward at the end of the latch section 2B so as to prevent the latch of the upper holding section 2D from coming off. By increasing the length of the upper holding section 2D, a plurality of drug solution containers can be suspended from and held on the container holder 2. Also, two or more upper holding sections 2D may be provided so that a drug solution container having two or more upper holding portions can be held.

An uneven portion is formed on the top surface of the upper holding section 2D as a displacement prevention mechanism that prevents the suspended drug solution container from displacing. With this formation, displacement of a plurality of drug solution containers after suspension from the upper holding section 2D can be reduced. Note that, while the uneven portion of the upper holding section 2D is shown as a corrugated portion in FIG. 1, the shape of the uneven portion is not limited to this, and can be formed of rectangles, triangles, etc. Moreover, not only the shape of the upper holding section, but the material thereof may be changed to form the prevention mechanism. For example, a mechanism of preventing displacement of a drug solution container may be provided by using a material resistant to sliding and using surface treatment.

The support 2A of the container holder 2 has a fixing section 2F for fixing the container holder 2 to the cover body 1. In the example in FIG. 1, the fixing section 2F is integrally formed with the support 2A. With this formation, by fixing the fixing section 2F of the container holder 2 to the cover body 1 by thermal welding, the inside of the exposure prevention cover can be put in the sealed state with the closure member to be described later.

Further, the fixing section 2F of the container holder 2 is integrally molded with a ring 2E as the suspension section permitting suspension from a drip stand. With this formation, the exposure prevention cover can be suspended from the drip stand with the ring 2E. While the container holder 2 is molded integrally including the ring 2E in this example, the formation is not limited to this. The ring for suspension from a drip stand may be formed as a separate member and be attached to the fixing section 2F fixed to the cover body 1 in a manner of sandwiching the fixing section 2F. Also, while one ring is provided in the example in FIG. 1, the invention is not limited to this, and two or more rings may be provided.

It is preferable that the container holder 2 be made of a plastic material having a strength enough to allow a plurality of drug solution containers to be suspended therefrom. Note however that the material is not limited to plastic but may be metal such as aluminum.

### (1-5 Needle Body 3)

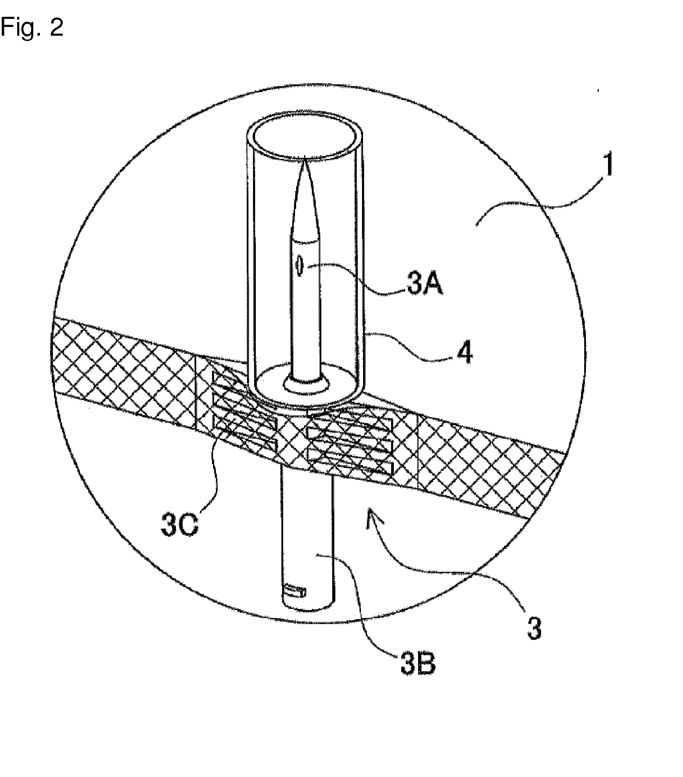

Next, the configuration of the needle body 3 provided with the needle cover 4 will be described with reference to the enlarged perspective view of FIG. 2. The needle body 3 constitutes the connection section connectable to the connection port of the drug solution container suspended in the exposure prevention cover. The needle body 3 includes a needle tip 3A, a protrusion tube 3B, and a fixing section 3C fixed to the cover body 1. The needle tip 3A is placed inside the cover body 1. The needle body 3 has a needle tip mouth near the center portion of the needle tip 3A in the length direction, and hollow space is formed inside from the needle tip mouth down to the bottom end of the protrusion tube 3B. The needle body 3 having this configuration can allow a drug solution such as an anticancer drug to pass therethrough.

The needle body 3 may be provided with an anti-falling structure in the needle tip 3A. For example, an rough surface may be formed, or the needle tip may have a conical shape in an upper portion from its tip by a given distance and then a narrow cylindrical shape in a lower portion down to its base. With such an anti-falling structure provided in the needle tip, the needle tip can be prevented from falling due to inadvertent pulling of a drug solution line, etc. after puncture of the connection port of the drug solution container.

The needle body 3 is made of a plastic material having a strength enough to be inserted into the connection port made of rubber, etc. of the drug solution container. The material is not limited to plastic, but may be metal such as stainless steel. A flexible needle may be used as a measure against mistaken puncturing of the cover body 1. For example, a structure where the base portion is in a boat shape and is coupled to the needle tip through an elastic body such as an elastic tube may be used.

### (1-6 Needle Cover 4)

The needle body 3 is provided with the needle cover 4 in a shape surrounding the needle tip 3A. The needle cover 4 has a shape of a cylinder open at the top and closed at the bottom, and approximately the center of the bottom surface is fixed to the base of the needle tip 3A. The needle cover 4 is formed to be longer than the height of the needle tip 3A. Thus, with the sharp tip of the needle body 3 covered with the cover body 1, the tip is blocked from coming into contact with the cover body 1, and thus an occurrence that the tip may stick into the cover body 1 and be damaged can be prevented. Also, in order to secure asepsis in the surroundings of the needle body 3, the top end of the needle cover 4 can be sealed to isolate the needle body 3 from the inside of the exposure prevention cover body 1.

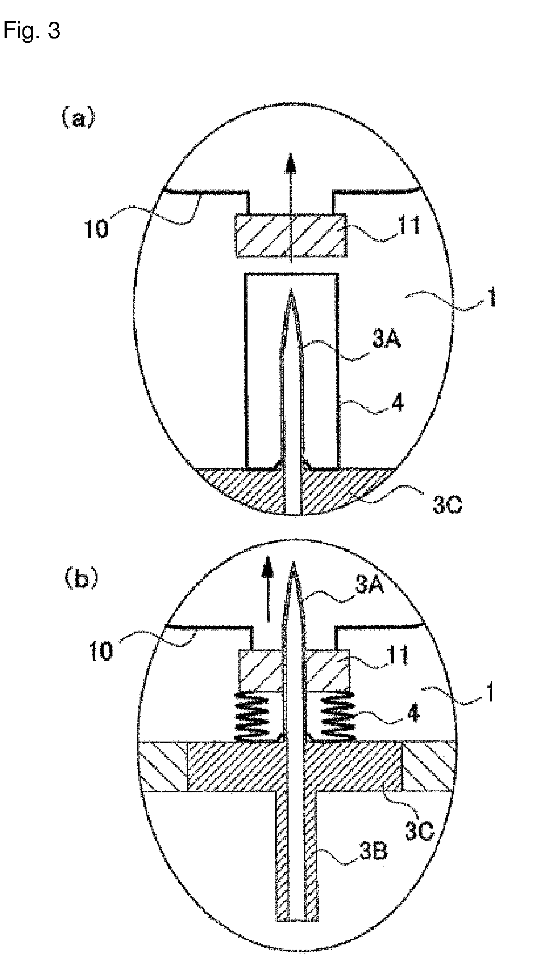

The state before insertion of the needle body 3 into the connection port of the drug solution container is shown in the enlarged cross-sectional view of FIG. 3(a), and the state where the needle body 3 has been inserted is shown in the enlarged cross-sectional view of FIG. 3(b). The needle cover 4 shown has such flexibility as to deform under an external force. Therefore, when the needle body 3 is inserted into the connection port of the drug solution container, the needle cover 4 deforms as shown in FIG. 3(b), contracting by being pressed between the connection port 11 of the drug solution container 10 and the needle tip 3C. In this way, while the needle cover 4 covers the needle tip 3A before use, it is deformed during use, not interrupting with the puncture of the connection port 11 of the drug solution container 10. Moreover, at the time when the needle tip 3C is pulled out from the connection port 11 of the drug solution container 10, the needle cover 4 expands recovering the state before use as shown in FIG. 3(a).

The needle cover 4 is formed of a flexible material such as silicone rubber. The material and the shape of the needle cover are not limited to those described above. For example, the needle cover 4 may have a shape of a bellows-like cylinder made of a plastic expandable in the length direction. Otherwise, the needle cover 4 may have a shape of a rectangular column, not a cylinder, or may have a shape where the diameter is not constant over the length, but the width is increased toward the tip of the needle body.

### (1-17 Method of Closing Exposure Prevention Cover)

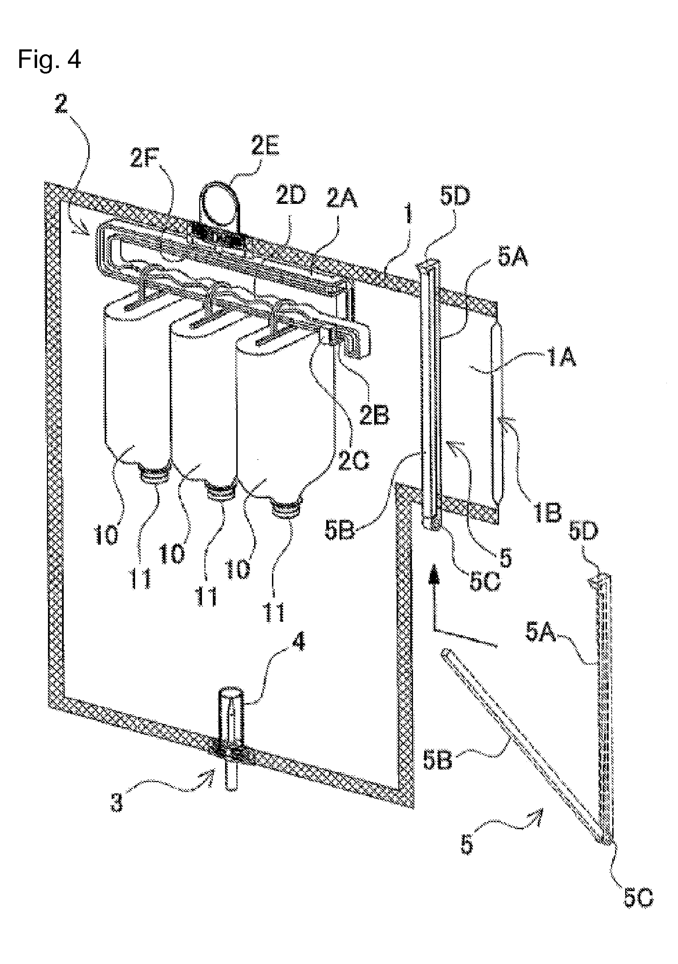

The state where drug solution containers are suspended in the exposure prevention cover and the opening is closed with a clip is shown as an external perspective view in FIG. 4. The drug solution containers 10 are inserted through the opening 1B of the cover body 1 and suspended from the upper holding section 2D. In the exposure prevention cover in FIG. 4, three drug solution containers 10 are suspended from the upper holding section 2D and the upper holding section 2D is latched on the latch section 2B and fixed. Thus, with the upper holding section 2D suspending three drug solution containers and being latched on the latch section 2B, the container holder 2 can stably hold the drug solution containers.

In the exposure prevention cover, also, after the suspension of the drug solution containers 10 in the cover body 1, the cover body 1 is closed with a clip 5 as the closure section that closes the protrusion 1A. The clip 5 is in a V-shape and includes mating members 5A and 5B, a rotation axis 5C at the bottom, and a joining part 5D. The clip 5 clips the protrusion 1A and mates the mating members 5A and 5B with each other by rotating the members around the rotation axis 5C. The joining part 5D on the mating member 5A is then latched on the mating member 5B in the mated state, whereby the clip 5 is fixed. By this closure, the exposure prevention cover can isolate the drug solution containers 10 inside from the external environment, permitting prevention of a damage due to exposure to the drug solution.

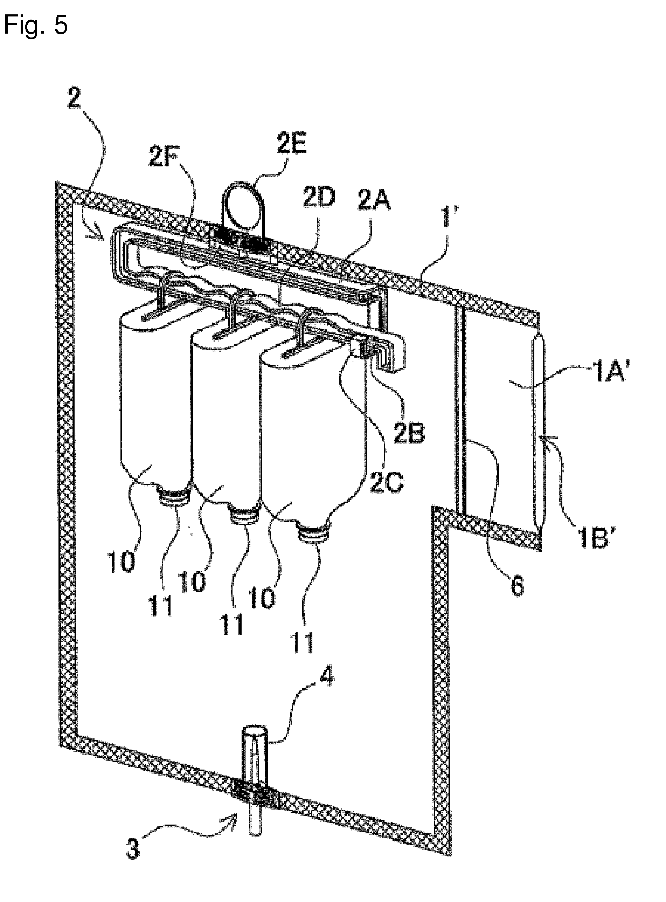

While the clip 5 is used as the closure section that closes the protrusion 1A in the above exposure prevention cover, the closure section is not limited to this. For example, a zipper seal may be used for closing, which is shown as an external perspective view in FIG. 5. In this exposure prevention cover, a zipper seal 6 where concavo-convex portions are engaged with each other is formed in advance in a protrusion 1A' of a cover body 1'. In the exposure prevention cover, the drug solution containers 10 are inserted through an opening 1B' and suspended from the upper holding section 2D. The zipper seal 6 as the closure section is then closed to permit sealing. By this closure, as in the case of the closure with the clip 5, the drug solution containers 10 inside can be isolated from the external environment, permitting prevention of a damage due to exposure to the drug solution. Moreover, the number of members required can be reduced by that for the clip 5. It is also possible to form a zipper seal on the right side face of the cover body 1' in advance without forming the protrusion 1A' of the cover body 1'.

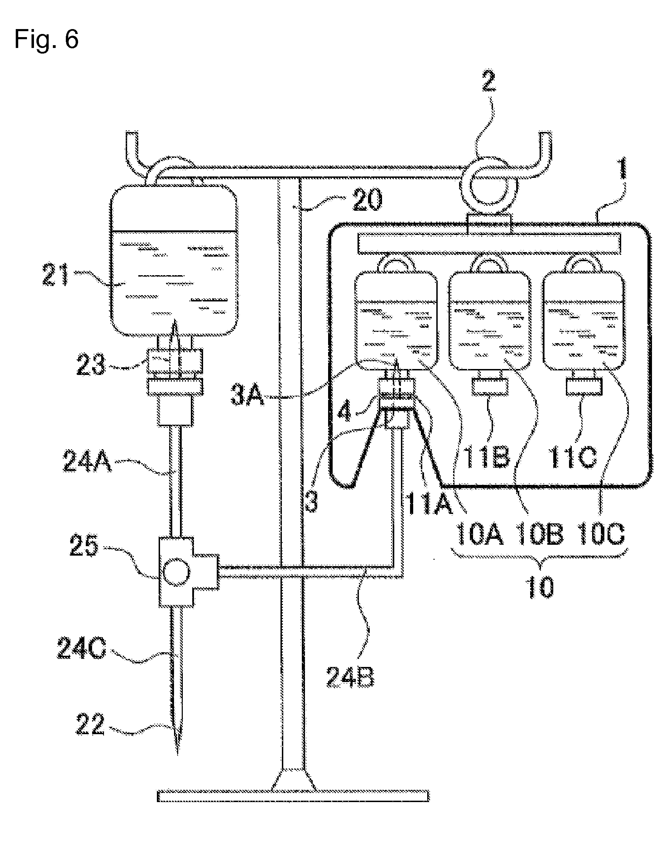

A primary use example of the exposure prevention cover will be described with reference to the schematic view of FIG. 6. In this example, an infusion container 21 filled with a normal saline solution and the exposure prevention cover in which drug solution containers 10A, 10B, and 10C are enclosed are suspended from a drip stand 20. During use, drug solution lines 24A, 24B, and 24C are connected to a three-way stopcock 25. A needle body 23 is connected to the other end of the drug solution line 24A, the needle body 3 is connected to the other end of the drug solution line 24B, and a puncture needle 22 is connected to the other end of the drug solution line 24C. Thereafter, the needle body 23 is inserted into the infusion container 21, and the needle body 3 of the exposure prevention cover is inserted into the drug solution container 10A. At this time, the three-way stopcock 25 is operated, to fill all the drug solution lines 24A, 24B, and 24C with the normal saline solution in the infusion container 21 thereby removing gas. The puncture needle 22 is then inserted into a human body, and the three-way stopcock 25 is further operated to administer the drug solution in the drug solution container 10A.

In the above use example, the drug solution containers 10 containing three kinds of anticancer drugs are suspended in the exposure prevention cover. In this illustration, the needle body 3 is inserted into a connection port 11A of the first drug solution container 10A out of the three containers. The needle cover 4 surrounding the needle body 3 is contracted at the time when the needle tip 3A is inserted into the connection port 11A of the first drug solution container 10A. Thus, the connection port 11A of the first drug solution container 10A and the needle tip 3A are in a state sealed by the needle cover 4, whereby leakage of the drug solution can be prevented.

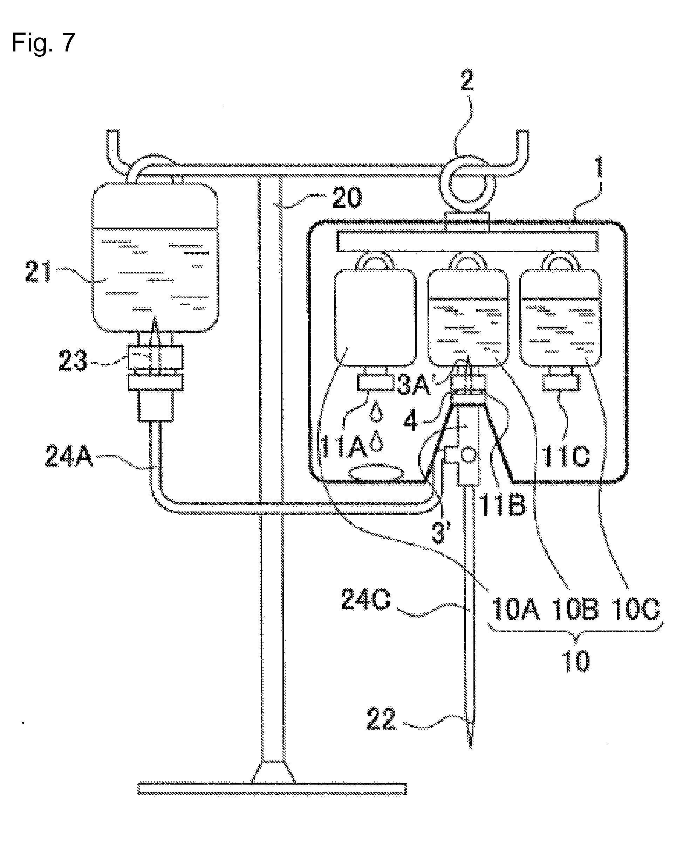

Next, a secondary use example of the exposure prevention cover to be executed after the primary use example will be described with reference to the schematic view of FIG. 7. The exposure prevention cover shown in FIG. 7 includes a needle body 3' provided with a three-way stopcock. The other configurations are similar to that in FIG. 6, and components corresponding to those in FIG. 6 are denoted by the same reference characters with detailed description thereof being omitted. FIG. 7 shows an example to be executed after the infusion of the first drug solution from the container 10A is completed. FIG. 7 shows the state where, in the cover body 1 of the exposure prevention cover, the needle body 3' has been removed from the connection port 11A of the first drug solution container 10A, and a needle tip 3A' of the needle body 3' is inserted into a connection port 11B of the second drug solution container 10B. It also shows the state where the drug solution is leaking from a minute hole of the connection port 11A of the first drug solution container 10A left after the removal of the needle body 3'. In this state, with the exposure prevention cover, the leaking drug solution can stay inside the cover body 1, preventing the solution from leaking outside the cover body 1. Likewise, although not shown, when the needle body 3' is removed after termination of administration of the second drug solution container 10B and then the needle tip 3A' of the needle body 3' is inserted into a connection port 11C of the third drug solution container 10C, a leaking drug solution from the connection port 11B of the second drug solution container 10B, if any, can stay inside the cover body 1, preventing the solution from leaking outside the cover body 1.

The exposure prevention cover according to this embodiment of the invention can be disposed of as medical waste with the drug solution containers kept enclosed inside at the time of completion of the infusion from all the drug containers. Therefore, exposure of the medical worker to the drug solutions at the disposal of the drug solution containers can also be avoided.

### (1-8 Absorbent)

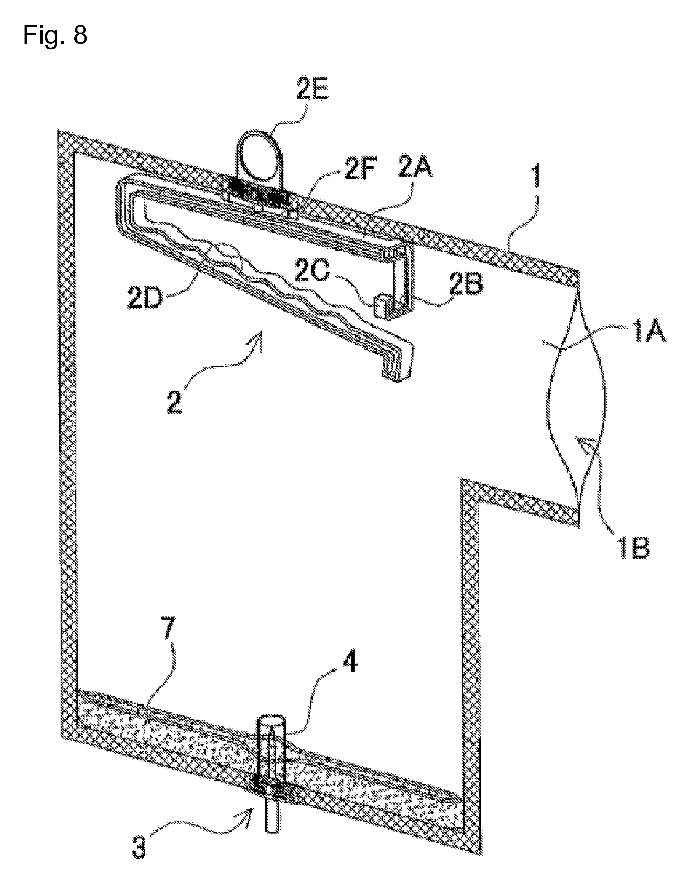

A water absorbent such as an absorption sheet may be provided in the bottom of the exposure prevention cover according to the invention. As an example, an exposure prevention cover provided with an absorbent is shown in the external perspective view of FIG. 8. The cover body 1 of this exposure prevention cover has an absorption sheet 7 inside in the bottom. The absorption sheet 7 can be formed of a multilayer structure of water absorption paper, a starch film, polyethylene, etc. It is preferable that the sheet is transparent, but even an opaque sheet can be used suitably by placing the sheet at a position where visual check of the inside of the exposure prevention cover is not hindered. With the absorption sheet 7 provided in the bottom of the cover body 1 as in FIG. 8, the transparency of the entire drug solution container can be secured even when an opaque absorption sheet is used. The cover body 1 can absorb a pool of the drug solution having leaked from the drug solution containers 10 with the absorption sheet 7. Moreover, even in an event of the cover body being damaged during handling at the disposal of the exposure prevention cover as medical waste, splashing of the drug solutions outside can be avoided because the drug solutions are held in the absorption sheet.

### <Second Embodiment>

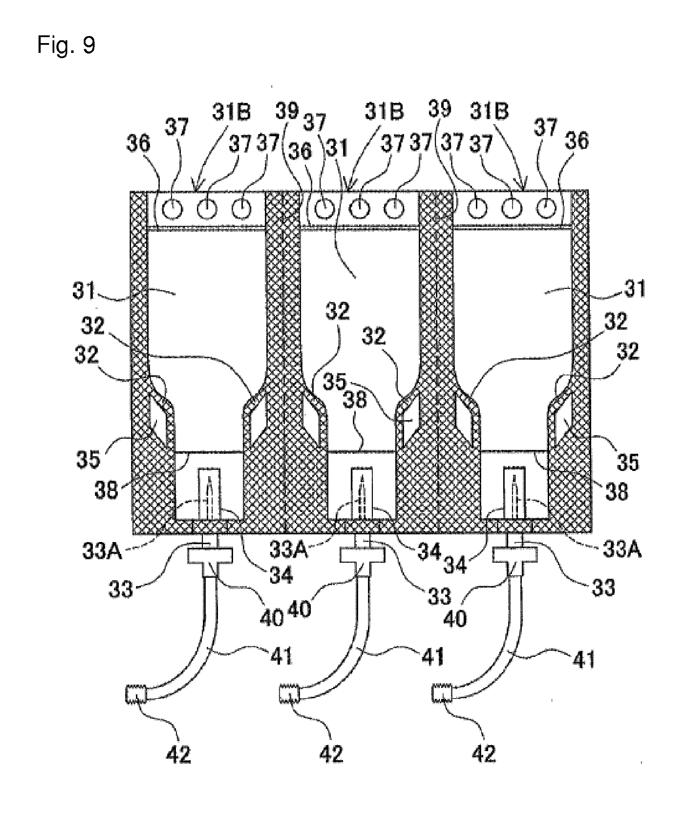

Next, an exposure prevention cover according to the second embodiment in which a container filled with a drug solution such as an anticancer drug is enclosed will be described with reference to the external front view of FIG. 9. This exposure prevention cover is comprised of a plurality of cover bodies 31 partitioned from each other so that drug solution containers can be stored individually. Note that, since the cover bodies 31 can be made of similar materials, etc. to those in the first embodiment, the following description will be made only on points unique to the second embodiment and detailed description on members in common with the first embodiment will be omitted.

### (2-1 Break Line 39)

The exposure prevention cover shown in FIG. 9 represents three cover bodies 31 coupled to one another. Each cover body is large enough to house only one drug solution container. The number of cover bodies 31 coupled is not limited to three, but a larger number of cover bodies 31 can be coupled depending on the amount and the number of kinds of drug solutions required. In such an exposure prevention cover, a plurality of cover bodies are coupled in advance into a sheet, and break lines 39 along which the cover bodies can be split apart are formed in the coupling portions. By breaking the sheet along a break line depending on the number of drug solution containers to be used, the number of cover bodies coupled can be adjusted freely. For example, when one drug solution container is used, one cover body 31 can be split apart along the break line 39 and used.

### (2-2 Simple partition 38)

The portion of each cover body 31 crosshatched in FIG. 9 is thermally welded. At the bottom of the portion, part of a needle body 33 is thermally welded. Inside the cover body 31, a needle tip 33A and a needle cover 34 integrated with the needle body 33 are enclosed. The cover body 31 is provided with an easily openable simple partition 38 at a position above the needle tip 33A and the needle cover 34. The simple partition 38 is formed as an easy peel-off seal by thermally welding the inner faces of the cover together. With this partition, the needle tip 33A and the needle cover 34 can be kept from being in contact with the outside air, maintaining an aseptic state, until the time of use of the exposure prevention cover. Note that, while the simple partition 38 is formed into a linear shape by partially welding the faces of the cover body together, it may be formed into a plane shape using another member. Note also that, although not shown, as described earlier, the welded portion may be made away from the periphery of the cover body, whereby the edge portion can be prevented from becoming rigid, to ensure that workability by the medical worker is not impeded.

The cover body 31 also has three suspension holes 37 used when being suspended from a drip stand, although the number of the suspension holes 37 is not limited to this. It also has a zipper seal 36 under the suspension holes 37 as the closure section for closing the cover body 31 after insertion of a drug solution container. Note however that, as in the first embodiment, a clip, etc. can also be used, instead of the zipper seal, as the closure section. Since the suspension holes 37 are required to support the weight of the entire drug solution container, it is preferable to reinforce the surroundings of the suspension holes 37. Although not shown, sheets may be additionally stuck on the front and back surfaces of a sheet constituting the portion of the suspensions holes, for example.

Next, the exposure prevention cover with a drug solution container inserted therein will be described with reference to the front view of FIG. 10. In the exposure prevention cover shown in FIG. 10, one drug solution container is housed in each of the three cover bodies 31 shown in FIG. 9. The cover body 31 has a lower holding section 32 as the holding section holding the drug solution container. In the illustrated example, the bottle-like drug solution container with its connection port protruding therefrom is supported from below by the lower holding section 32. That is, as shown by crosshatching in FIG. 10, when two sheets constituting the cover body 31 are thermally welded together to form the storage space for the drug solution container, a lower welded portion is made wide while a middle and upper welded portion is made narrow. In other words, the storage space for the drug solution container is formed to be narrow in a lower portion and wide in an upper portion. The narrow space for the drug solution container serves as the lower holding section 32. The opening of the lower holding section 32 has such a width that allows insertion of the connection port portion of the drug solution container but does not allow insertion of the entire drug solution container because the container hits the wall at its shoulder. Therefore, the shoulder portion of the drug solution container near the connection port can be supported with the lower holding section 32, permitting holding of the drug solution container in the cover body 31. Since such a lower holding section 32 can be formed from the cover body, it can be formed at low cost without the necessity of any additional member as that of the upper holding section described above. Also, with the lower holding section 32, any sized drug solution containers different in quantity can be held at their shoulders as far as the sizes of the protruding connection ports are approximately the same. By inclining the abutting portion of the lower holding section 32 that abuts against and supports the shoulder of a container, the lower holding section 32 can hold drug solution containers of various sizes stably. It is however needless to mention that holding a container is also possible with a straight-line abutting portion.

In the exposure prevention cover, a three-way stopcock 40 is connected to the needle body 33 of each of the three cover bodies 31 coupled to one another. An intermediate tube 41 is connected to the bottom of the three-way stopcock 40. A coupling connector 42 is connected to the other end of the intermediate tube 41. In FIG. 10, the three-way stopcock 40 of each of the three cover bodies 31 is connected to an adjacent three-way stopcock 40 via the intermediate tube 41 provided with the coupling connector 42. Also, although not shown, the coupling connector 42 on the left in FIG. 10 is connected to a main drug solution line.

### (2-3 Connection to Drug Solution Container)

After the connection of the coupling connectors 42, drug solutions in drug solution containers 50 can be sequentially fed out by connecting the needle tip 33A of each needle body 33 to a connection port 51 of the corresponding drug solution container 50. It is preferable that the needle body 33 be provided with a check value to prevent backflow of the solution to the drug solution container 50. The three-way stopcocks 40 and the coupling connectors 42 are color-coded to prevent wrong coupling.

The cover body 31 is provided with grasp holes 35 formed at two positions with which the circumference of the connection port 51 of the drug solution container 50 can be grasped from outside. With this configuration, the medical worker can insert fingers through the grasp holes 35 to grasp the circumference of the connection port 51 of the drug solution container 50 with the fingers. As a result, the medical worker can pinch the needle body 33 with fingers of the other hand and press the needle body 33 into the connection port 51 fixed with the fingers, whereby puncture with the needle tip 33A can be performed reliably. Note that, while the shape of the grasp holes 35 is rhombic in the illustrated example, it is not limited to this. It can be rectangular or elliptic as far as fingers can be inserted therethrough.

As described above, according to this embodiment, where one cover is used for one drug solution container, exposure to the drug solution can be effectively prevented. For example, while a large cover is necessary in preparation for an increase in the number of drug solution containers in the case of the cover in FIG. 1, this problem can be solved by using one cover for one drug solution container as described above.

### (2-4 Variation)

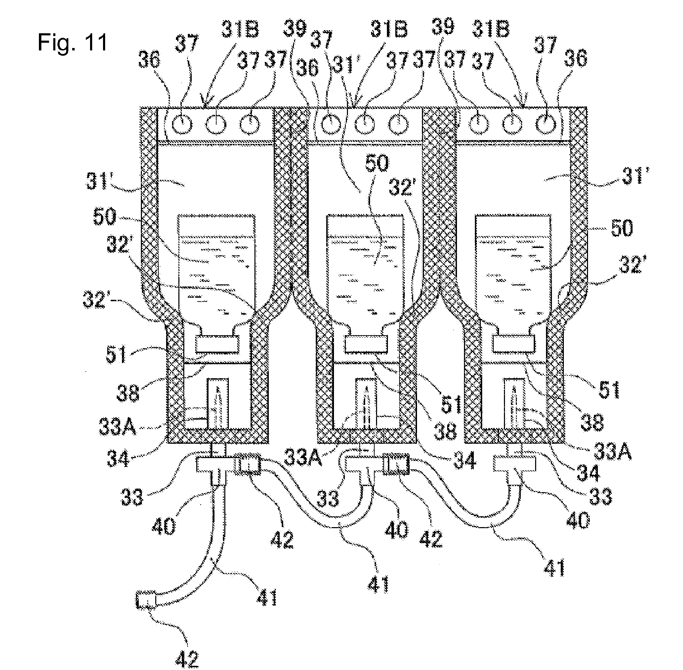

FIG. 11 shows a cover body 31' of a variation where the circumference of the connection port 51 of the drug solution container 50 can be grasped with fingers without provision of the grasp holes 35. In this cover body 31', as in the cover body 31 described above, a lower holding section 32' that holds the drug solution container 50 near the connection port 51 is formed at two positions. The cover body 31' can be formed so that the outer shape of the portion thereof below the lower holding section 32' is narrow in advance. With this shape, the circumference of the connection port of the drug solution container housed in the cover body 31' can be grasped and held with a hand from outside.

The cover body 31 in the second embodiment is not limited to that described above. For example, the needle tip 33A and the needle cover 34 may be connected from outside the cover body. Also, the lower welded portion may be formed into a pouch shape so that the lower portion of the cover body is soft while the upper portion thereof is rigid. By using such forms, while the cover body is kept fixed, the needle tip 33A and the connection port 51 of the drug solution container can be connected to each other. This improves the operability.

### <Third Embodiment>

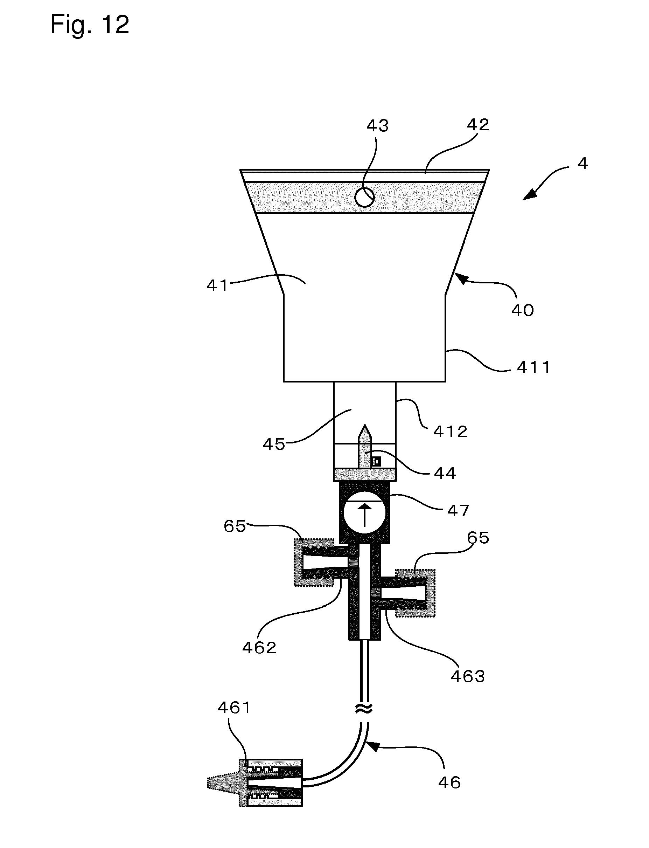

The third embodiment of the present invention will be described with reference to FIG. 12. FIG. 12 is a front view of a cover module according to this embodiment.

### (3-1 Cover Module)

As shown in FIG. 12, a check value 47 and an intermediate tube 46 to be described later are attached to an exposure prevention cover 40 of this embodiment, and the exposure prevention cover 40 is used as a cover module 4 including these attachments. The exposure prevention cover 40 includes a cover body 41 having an opening at the top, and is configured so that the opening can be closed with a slidable zipper seal 42. That is, the opening can be closed by moving a slide member of the zipper seal. The top opening of the cover body 41 is large in diameter, and a lower portion 411 thereof has such an inner diameter so as to fit to the outer shape of a drug solution container to be housed. With this configuration, the drug solution container inserted from the top opening of the cover body 41 is fixed in the lower portion of the cover body 41, and thus held with the surface of the drug solution in the drug solution container being kept horizontal. This form of the lower portion 411 of the cover body 41 corresponds to the holding section according to the present invention. Thus, the remaining quantity of the drug solution can be visually recognized correctly. A suspension hole 43 is formed in an upper portion of the cover body 41.

A small-diameter insertion section 412 in which the connection port of the drug solution container is inserted is provided in a lower portion of the cover body 41. The insertion section 412 has a cylindrical shape protruding from the bottom of the cover body 41. The insertion section 412 is provided with a needle body 44 and a needle cover 45 having functions similar to those described in the second embodiment. The needle cover 45 may be configured to extend upward from the base end of the needle body as shown in FIG. 3, or configured to extend downward to cover the needle body 44 from the top end of the insertion section 412 as shown in FIG. 12. The needle body 44 is provided with a communication port (not shown) that communicates with an inner passage of the needle body 44 formed at a position near the base end that is not inside the connection port 11A of the drug solution container 10A when the needle body is inserted into the connection port 11A. The communication port is configured to communicate with the inside of the cover body 41, whereby, when the drug solution is discharged from the drug solution container 10A, air in the cover body 41 is allowed to flow into the drug solution container 10A via the communication port through the inside of the needle body 44. As a result, the drug solution can be easily discharged from the drug solution container 10A.

The intermediate tube 46 (drug solution line, drug solution discharge line) is coupled to the needle body 44 via the check valve 47. As the check valve 47, a known backflow prevention float-type one-way valve, for example, can be used to perform priming to be described later. With such a value, it is possible to ensure that, while air and a liquid can flow from the cover body 41 to the intermediate tube 46, a liquid is not allowed to flow, although air is allowed to flow, from the intermediate tube 46 to the cover body 41. A connection adapter 461 having a thin film cover (not shown) is attached to the tip opening of the intermediate tube 46. As far as this cover is not broken, the drug solution flowing in the intermediate tube 46 is prevented from flowing from the tip opening. Alternatively, a lock mechanism that will not be disengaged once being engaged can be attached. The intermediate tube 46 is provided with a pair of ports near the check valve 47: a flushing port 462 from which a normal saline solution is injected provided at a position closer to the check valve 47, and a coupling port 463 for coupling an intermediate tube 46 for another exposure prevention cover at a position away from the check valve 47.

As will be described later, a syringe (infusion solution injection member) for injecting a normal saline solution is coupled to the flushing port 462. Inside the port 462, placed is a check valve that opens by an injection pressure when the tip of the syringe is coupled to the port 462 and the normal saline solution is injected. Inside the coupling port 463, placed are a check valve that opens with insertion of another intermediate tube and a hollow needle body. With this configuration, when the tip of another intermediate tube is inserted into the coupling port, the cover is broken to allow a drug solution to flow from this intermediate tube into the coupling port 463. Note that, when a drug solution is injected from the coupling port 463, flow of the drug solution into the exposure prevention cover 40 via the needle body 44 is cut off by the action of the check valve 47. Each of the flushing port 462 and the coupling port 463 is closed with a lid member 65 before use, and the lid member 65 is removed for use.

### (3-2 Infusion Container and Accessories)

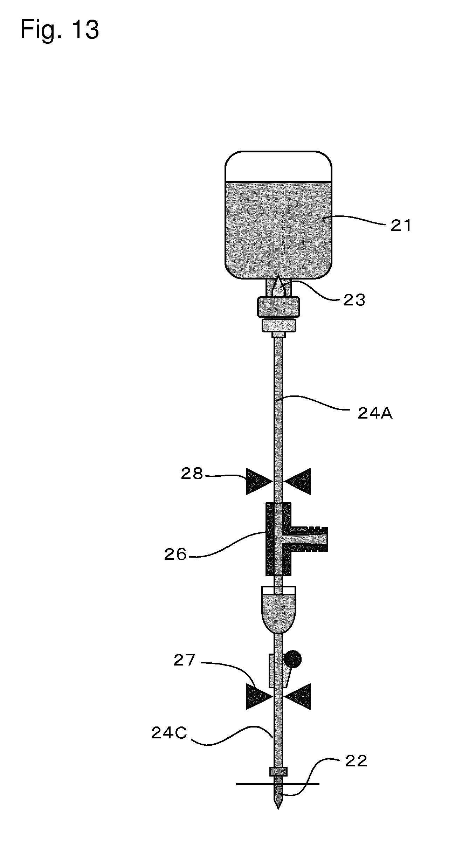

Next, an infusion container 21 containing an infusion solution such as a normal saline solution will be described with reference to FIG. 13. FIG. 13 is a front view of a drug solution container for a normal saline solution and its accessories according to this embodiment. The infusion container 21 as used herein is approximately the same as that shown in FIG. 6 in the first embodiment. The point different from the first embodiment is the configuration of a three-way stopcock 26 (coupling section) as shown in FIG. 13, to which the connection adapter 461 at the tip of the intermediate tube 46 described above can be attached. That is, a hollow needle body (not shown) is attached to the three-way stopcock 26 so that the thin film cover of the connection adapter 461 can be broken when the intermediate tube 46 is attached thereto.

### (3-3 Method of Supplying Drug Solution)

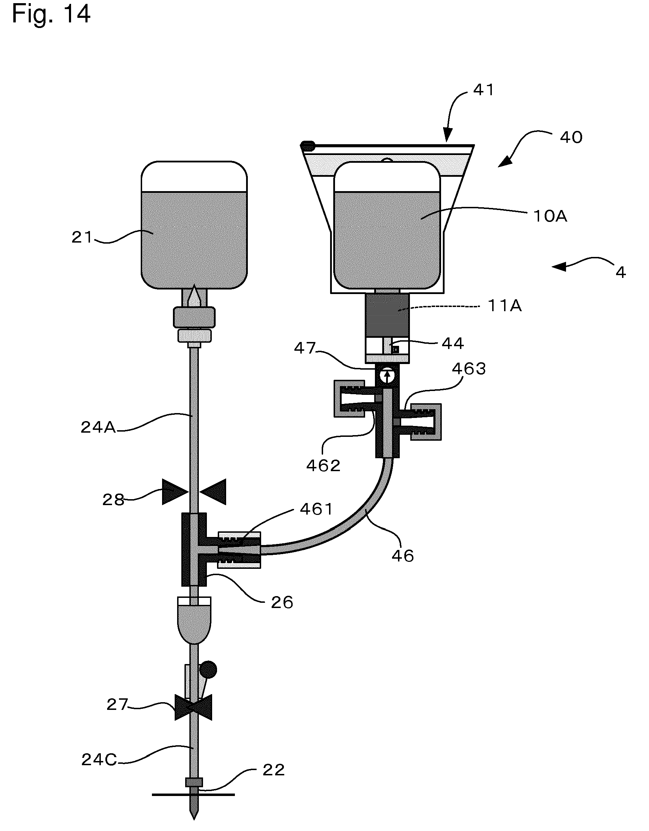

Next, a method of supplying a drug solution using the exposure prevention cover 40 configured as described above will be described with reference to FIGS. 14 to 18. First, drug solution containers 10A and 10B containing drug solutions of the number required and cover modules 4 and 5 for housing the drug solution containers are prepared. The drug solution containers 10A and 10B are housed in exposure prevention covers 40 and 50 of the cover modules 4 and 5 and sealed. The drug solution containers 10A and 10B are held on lower portions 411 and 511 of the exposure prevention covers 40 and 50, and fixed in the covers so that the liquid surface is horizontal. At this time, care should be taken to keep needle bodies 44 and 54 of the exposure prevention covers 40 and 50 from sticking into connection ports 11A and 11B of the drug solution containers 10A and 10B. Herein, an example of using two drug solution containers 10A and 10B will be described, referring to the cover module connected first as the first cover module 4 and the cover module connected secondly as the second cover module 5 (additional module).

First, the procedure of supplying the drug solution in the first cover module 4 will be described. In the state shown in FIG. 13, the infusion solution is discharged from the infusion container 21 to be discharged from the puncture needle 22 through an upstream drug solution line 24A (infusion solution discharge line), the three-way stopcock 26, and a downstream drug solution line 24C (supply line). This removes air in the drug solution lines 24A and 24C (first priming). In this state, the puncture needle 22 is inserted into a patient. Thereafter, a first clip 27 (control member) is fastened to the downstream drug solution line 24C, to temporarily block administration of the infusion solution to the patient. Note that any member other than the clip may be used as far as the member can cut off flow of a drug solution. Subsequently, as shown in FIG. 14, the intermediate tube 46 of the first cover module 4 is connected to the three-way stopcock 26. Since the three-way stopcock 26 is provided with the needle body as described above, when the tip of the intermediate tube 46 is connected to the three-way stopcock 26, the cover of the connection adapter 461 is broken, allowing communication between the three-way stopcock 26 and the intermediate tube 46. Thus, the infusion solution flows from the infusion container into the intermediate tube 46. As a result, gas inside the intermediate tube 46 is pushed toward the exposure prevention cover 40 by the infusion solution, and flows into the cover body 41 via the check valve 47. Note however that, with the check valve 47, the infusion solution is prevented from flowing into the cover body 41. In this way, the intermediate tube 46 is filled with the infusion solution, removing the gas (second priming).

Thereafter, a second clip 28 (control member) is fastened to the upstream drug solution line 24A that is connected to the infusion container 21, to block the communication of the infusion solution. That is, the flow of the normal saline solution from the infusion container 21 to the three-way stopcock 26 is cut off. Subsequently, in the exposure prevention cover 40, the needle body 44 is inserted into the connection port 11A of the drug solution container 10A to allow the drug solution to flow into the intermediate tube 46. In this state, by releasing the first clip 27, the drug solution is administered to the patient via the intermediate tube 46, the three-way stopcock 26, and the downstream drug solution line 24C.

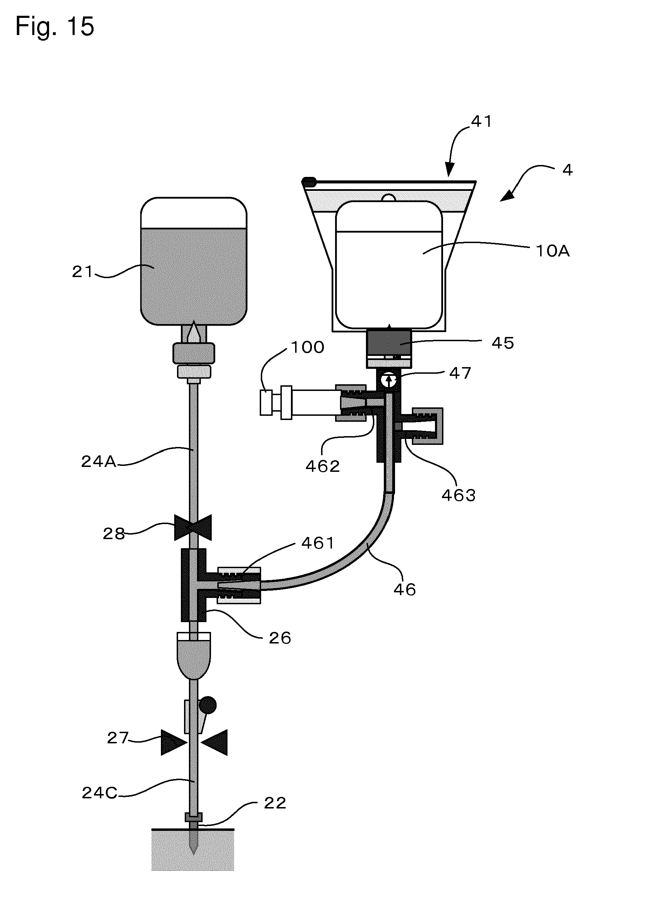

Once the remaining quantity of the drug solution in the drug solution container 10A becomes zero, the cover 65 of the flushing port 462 provided on the intermediate tube 46 is removed, and a syringe 100 with a normal saline solution injected therein is attached to the port 462, as shown in FIG. 15. A piston of the syringe 100 is pushed in to inject the normal saline solution from the flushing port 462 into the intermediate tube 46. The normal saline solution is prevented from entering the inside of the exposure prevention cover 40 by the check valve 47, and flows toward the three-way stopcock 26. By this flowing, the drug solution remaining in the intermediate tube 46 is pushed out with the normal saline solution, to be administered to the patient (flushing). Once the injection of the normal saline solution is completed, the syringe 100 is removed.

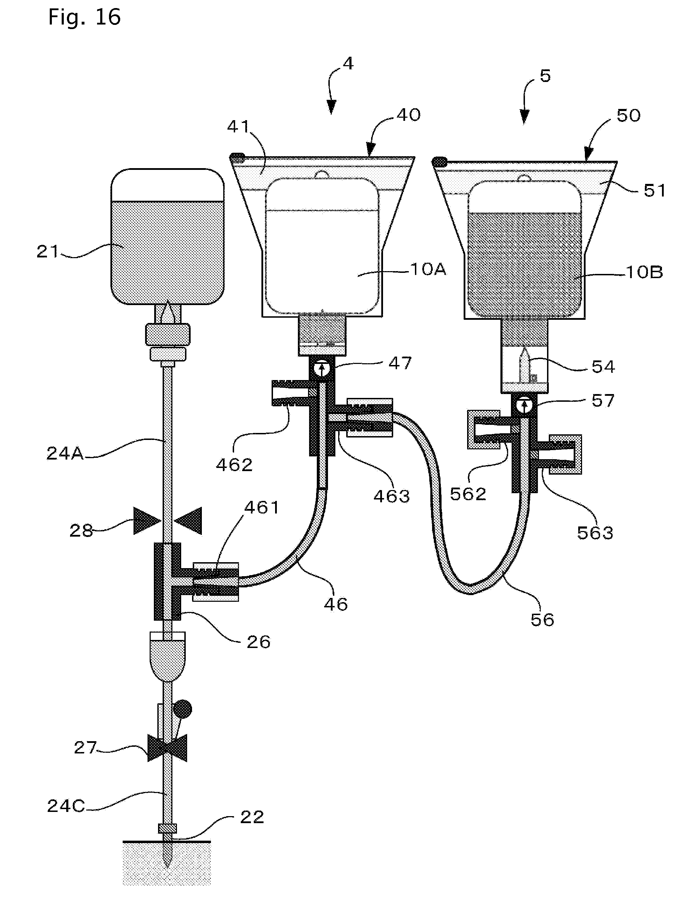

Next, the drug solution in the second cover module 5 is supplied. First, the downstream drug solution line 24C is blocked with the first clip 27. Subsequently, as shown in FIG. 16, an intermediate tube (additional drug solution discharge line, additional discharge line) 56 of the second cover module 5 is connected to the coupling port 463 of the intermediate tube 46 of the first cover module 4. By this connection, the intermediate tubes 46 and 56 of the cover modules 4 and 5 communicate with each other. In this state, the second clip 28 is released to discharge the infusion solution from the infusion container 21. The infusion solution flows into the intermediate tube 56 of the second cover module 5 via the three-way stopcock 26 and the intermediate tube 46 of the first cover module 4. By this flowing, gas inside the intermediate tube 56 is pushed into the exposure prevention cover 50 (second priming). Once the intermediate tube 56 is degased, the second clip 28 is fastened, and, in the second cover module 5, the needle body 44 is inserted into the connection port 11B of the drug solution container 10B. Subsequently, by releasing the first clip 27, the drug solution discharged from the drug solution container 10B is administered to the patient via the two intermediate tubes 46 and 56, the three-way stopcock 26, and the downstream drug solution line 24C.

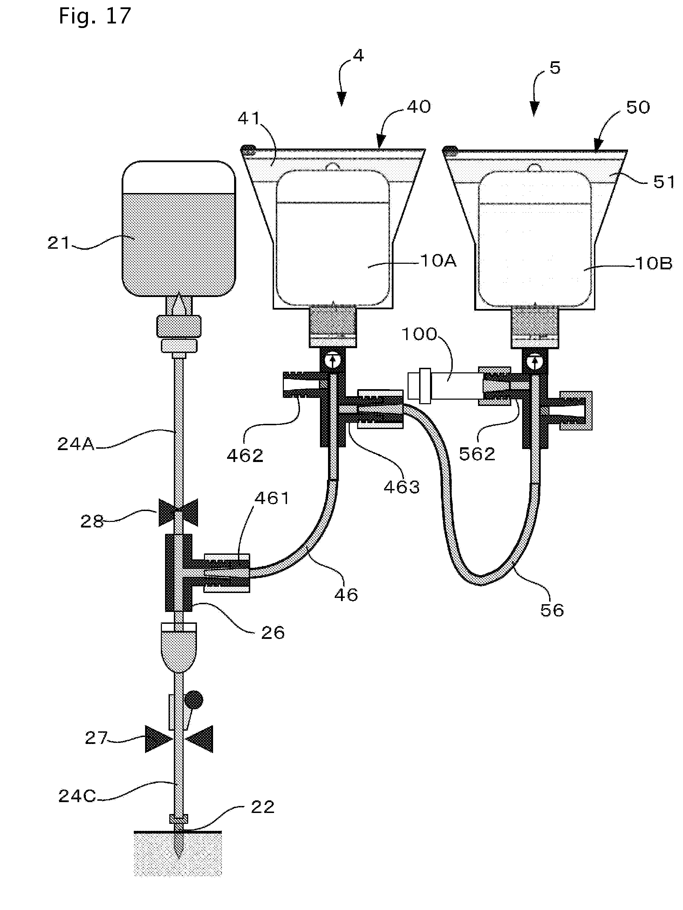

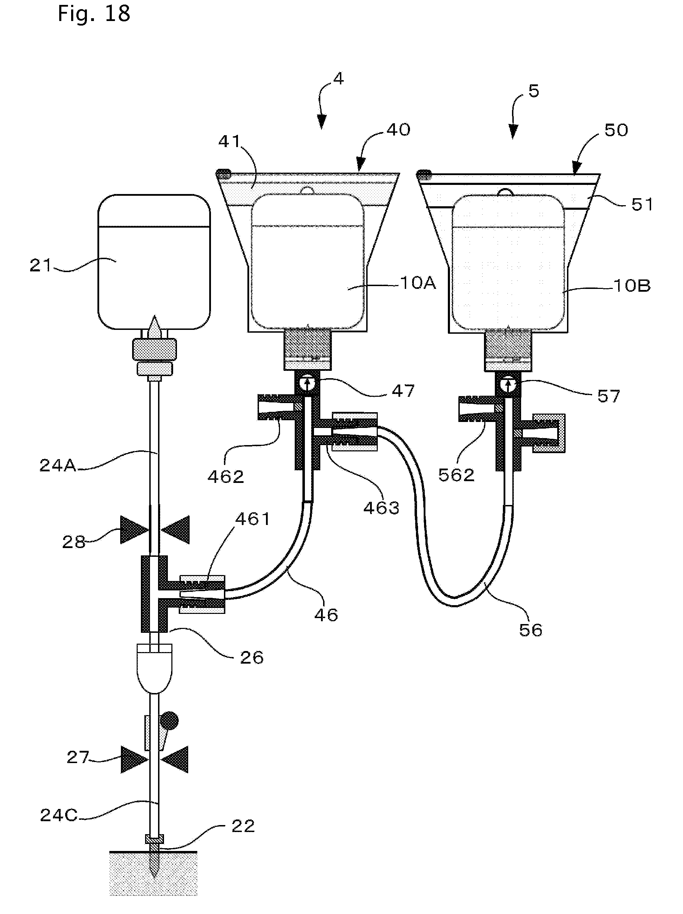

As was done in the first cover module 4, once the drug solution in the drug solution container becomes zero, the syringe 100 is attached to a flushing port 562 of the second cover module 5, to inject the normal saline solution into the intermediate tube 56, as shown in FIG. 17. By this injection, the drug solution remaining in the intermediate tube 56 is pushed out to be administered to the patient. Finally, as shown in FIG. 18, the first and second clips 27 and 28 are released to administer the remaining infusion solution in the infusion container 21 to the patient. Once the administration of the drug solution is completed, the puncture needle 22 is removed from the patient. Thereafter, all of the infusion container 21, its accessories 24A, 26, and 24C, and the first and second cover modules 4 and 5 are disposed of.

As described above, according to this embodiment, as in the first and second embodiments, it is possible to administer a plurality of drug solutions while preventing exposure to the drug solutions. Also, in administering a plurality of drug solutions, by preparing a plurality of cover modules 4 and 5 and serially connecting their intermediate tubes 46 and 56 to each other, the drug solutions can be administered through one route without the necessity of administering the drug solutions individually from the cover modules 4 and 5. Priming can also be performed using this route. Moreover, by providing the flushing ports 462 and 562 on the intermediate tubes 46 and 56 to inject a normal saline solution, all of the intended amounts of the drug solutions can be administered to the patient. Thus, the correct amount of a drug solution can be administered.

### (3-4 Variations)

While two cover modules are used in this embodiment, the number of cover modules is not specifically limited. The configurations of the intermediate tubes and the check valves are not specifically limited, but known members can be appropriately used. When three or more cover modules are used, their intermediate tubes should be serially connected. That is, as described above, the tip end of one intermediate tube should be connected to the base end of another intermediate tube, i.e., to the coupling port near the exposure prevention cover, to be arranged so that drug solutions in all drug solution containers flow into the three-way stopcock through one route.

While the check valve 47 is provided in the cover module described above, the invention is not limited to this, and it is only necessary to provide a backflow prevention member for preventing the drug solution discharged from the drug solution container 10A to the intermediate tube 46 from flowing backward to the exposure prevention cover 41. Examples of such a backflow prevention member include a clip and a drip chamber. When the infusion solution is supplied to the intermediate tube 46 during priming, air moves toward the exposure prevention cover 41. In order to remove this air from the intermediate tube 46, the clip may be released/fastened while backflow of the solution being prevented by visual check. In this way, only air can be allowed to enter the exposure prevention cover 41. Also, using the drip chamber, the air can be stored inside the drip chamber.

While the clips are used as the control members 27 and 28 that control the flow of the solution in the lines 24A, 24C, 46, and 56 in the above-described embodiments, the invention is not limited to this. For example, members such as roller clamps that press the line to cut off the flow of the solution may be used. Also, mechanical and electrical valve mechanisms may be used to control the flow of the solution.

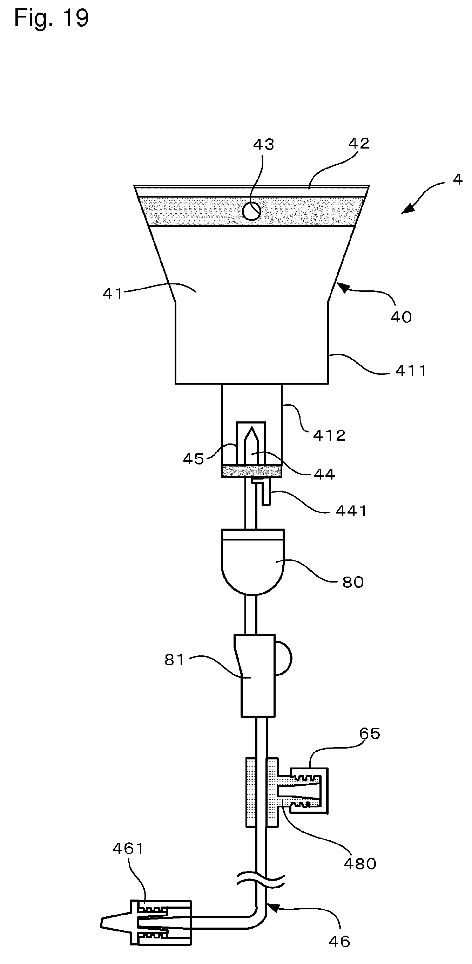

An example of the cover module different from that in FIG. 12 is shown in FIG. 19. In this example, a drip chamber 80 is used as the backflow prevention member and a roller clamp 81 is used as the control member, and these are attached to the intermediate tube 46 in this order from the side close to the cover 41. A port 480 serving as both the flushing port and the coupling port is attached at a position downstream of the roller clamp 81. As the needle body 44, a known two-hole needle provided with an air induction section 441 is used, to be adaptable even when the drug solution container 10A is a rigid bottle. The air induction section 441 is provided with a hydrophobic filter and a check valve to avoid leakage of the drug solution to the outside. The needle body 44 is provided with a tube-shaped needle cover 45. The method of using this cover module is basically the same as those in the above embodiments, except that, during the first and second priming, the roller clamp 81 is operated while recognizing removal of gas in the intermediate tube 46 by visual check and that, since one port is used as both the flushing port and the coupling port, the intermediate tube 56 of the second cover module 50 is coupled to the same port 480 from which the syringe has been removed after termination of the flushing operation.

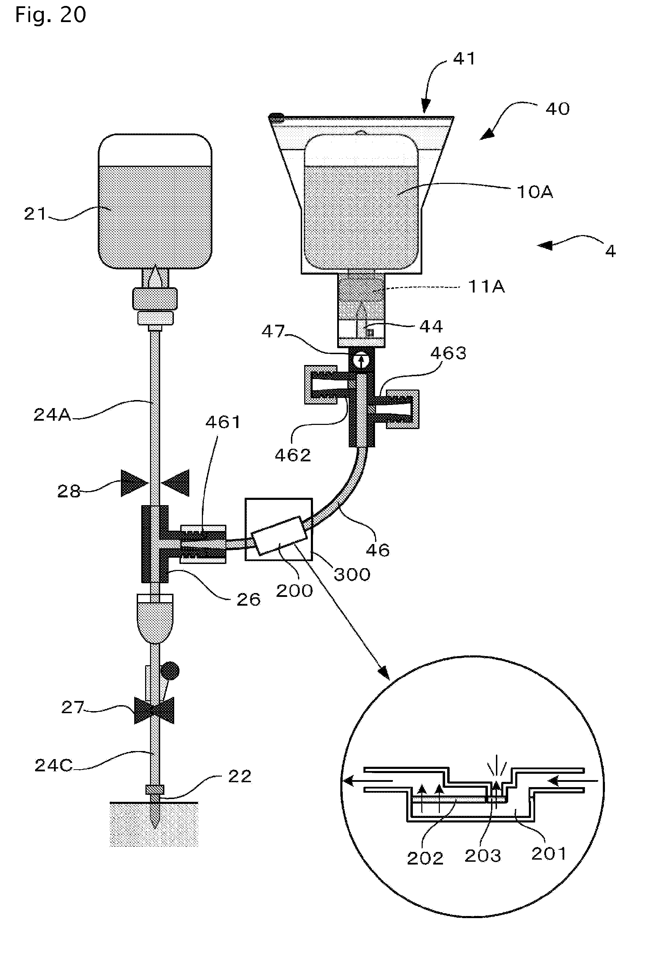

In the third embodiment, priming of the intermediate tube 46 is performed by supplying the infusion solution from the infusion container 21 to the intermediate tube 46. In this relation, a configuration as shown in FIG. 20 may be used. In this example, an air discharge filter 200 is provided in a downstream portion of the intermediate tube 46. As such an air discharge filter 200, a known filter configured to discharge air outside while allowing a liquid to pass through can be used. While the air discharge filter 200 can have any of various configurations, it may be configured as shown in an enlarged view in FIG. 20, for example, where a hydrophilic film 202 and a hydrophobic film 203 are provided in a flow path 201 in the filter 200. The hydrophilic film 202 partitions the intermediate tube 46 into an upstream part and a downstream part, and the hydrophobic film 203 serves as a partition between the flow path 201 in the filter 200 and the outside. Thus, the solution flows toward the downstream part of the intermediate tube 46 through the hydrophilic film 202, and gas is discharged outside through the hydrophobic film 203. In this way, it is possible to prevent air from being discharged into the drug solution line 24C from the intermediate tube 46 without performing priming. Moreover, once the drug solution is exhausted, the flow stops, and the flow of the solution becomes slow with the hydrophilic film 202. Further, the air discharge filter 200 is sealed with a container (air collection container) 300 such as a bag. That is, since there is the possibility that the air discharged through the hydrophobic film 203 may have been altered by the drug solution, such air is sealed inside the container 300 to prevent exposure.

In place of using such an air discharge filter 200, a known air removal filter through which air does not pass may simply be provided in the intermediate tube 46. This filter allows passage of only a liquid and does not allow passage of air. Therefore, air stays on the upstream side of the filter.

The configuration of the exposure prevention cover used in the third embodiment is not specifically limited, and that used in the first or second embodiment can be used. As for the other configuration, those in the first to third embodiments can be combined appropriately. Design changes such as placing the zipper seal 42 on a side face of the cover body 41 can be made appropriately depending on the needs.

While the case of using a medical solution such as an anticancer drug was described in the above embodiments, the invention is not limited to this, and can be applied to any drug solutions to which exposure should be prevented as a whole.

### [Industrial Applicability]

The exposure prevention cover, the exposure prevention cover module provided with the same, the drug solution supply system, and the drug solution supply method according to the present invention can be suitably used for the prevention of exposure to a drug, etc. For example, they can be suitably used for the prevention of exposure to a drug such as an anticancer drug that has a strong effect.

## Claims

1. An exposure prevention cover for housing a drug solution container that contains a drug solution and has a connection port through which the drug solution can be discharged, comprising:
a cover body that houses the drug solution container and has an opening open to the outside for inserting the drug solution container;
a closure section that closes the opening;
a holding section that holds the drug solution container housed in the cover body inside the cover body;
a connection section that is attached to the cover body and can be connected to the discharge port of the drug solution container, so that the drug solution in the drug solution container can be discharged outside the cover body; and
a suspension section provided on the cover body for suspending the cover body.

2. The exposure prevention cover of claim 1, wherein the cover body is made of a material having at least one of a light transmittance property, a gas barrier property, a waterproof property, and a light shielding property.

3. The exposure prevention cover of claim 1 or 2, wherein the closure section is comprised of a clip that fastens the opening from both sides.

4. The exposure prevention cover of claim 1 or 2, wherein the closure section is comprised of a zipper seal capable of closing the opening.

5. The exposure prevention cover of any of claims 1 to 4, wherein at least part of the cover body has a combination of materials having a water absorption property.

6. The exposure prevention cover of any of claims 1 to 5, wherein at least one said suspension section that suspends the cover body with the drug solution container housed therein is provided on an upper part of the cover body.

7. The exposure prevention cover of any of claims 1 to 6, wherein the cover body has a volume capable of housing only one drug solution container.

8. The exposure prevention cover of claim 6 or 7, wherein the holding section is a lower holding section having an opening size that allows insertion of the connection port protruding from the drug solution container housed in the cover body but does not allow insertion of a shoulder of the drug solution container near the connection port.

9. The exposure prevention cover of any of claims 1 to 8, wherein the holding section is a container holder that engages with a hanging hole formed on the drug solution container in the cover body so as to be able to suspend the drug solution container.

10. The exposure prevention cover of claim 9, wherein the container holder has an upper holding section that is inserted through the hanging hole formed on the drug solution container so as to be able to suspend the drug solution container, and
the top surface of the upper holding section is formed so as to be uneven.

11. The exposure prevention cover of any of claims 1 to 10, wherein the connection section further includes
a needle body for being inserted into the connection port of the drug solution container, and
a needle cover that has a cylindrical shape surrounding the needle body and extends longer than the height of the needle body.

12. The exposure prevention cover of claim 11, wherein the needle body is provided with an anti-falling structure for preventing the needle body from falling after being inserted into the connection port of the drug solution container.

13. The exposure prevention cover of claim 11 or 12, wherein the needle cover is made of a flexible material.

14. The exposure prevention cover of any of claims 1 to 13, wherein a simple partition that seals the needle body and the needle cover in the cover body is provided.

15. An exposure prevention cover module comprising:
the exposure prevention cover of any of claims 1 to 14;
a backflow restriction member that is attached to the connection section of the exposure prevention cover to restrict backflow of a liquid; and
a drug solution discharge line attached to the connection section via the backflow restriction member.

16. The exposure prevention cover module of claim 15, wherein at least one port via which a liquid can be injected is provided in the drug solution discharge line.

17. A drug solution supply system comprising:
the exposure prevention cover of any of claims 1 to 14;
at least one drug solution container housed in the exposure prevention cover;
an infusion container containing an infusion solution;
an infusion solution discharge line connected to the infusion container at one end to discharge the infusion solution from the infusion container;
a drug solution discharge line connected to the connection section of the exposure prevention cover at one end to discharge a drug solution in the drug solution container housed in the exposure prevention cover;
a coupling section that couples the other end of the drug solution discharge line and the other end of the infusion solution discharge line;
a supply line connected to the coupling section to discharge a liquid from at least one of the infusion solution discharge line and the drug solution discharge line; and
a control member that controls flow of a liquid in at least one of the lines.

18. The drug solution supply system of claim 17, further comprising an infusion solution injection member attached to a portion of the drug solution discharge line near a junction with the connection section to inject an infusion solution into the drug solution discharge line.

19. The drug solution supply system of claim 17 or 18, further comprising an air removal filter that is attached to the drug solution discharge line and allows passage of the drug solution flowing from the drug solution container but does not allow passage of air.

20. The drug solution supply system of claim 17 or 18, further comprising:
an air exhaust filter that is attached to the drug solution discharge line and allows passage of the drug solution flowing from the drug solution container but exhausts air outside; and
an air collection container that seals air exhausted from the air exhaust filter.

21. The drug solution supply system of any of claims 17 to 20, further comprising an additional module that includes
at least one exposure prevention cover of any of claims 1 to 14,
a drug solution container housed in each of the at least one exposure prevention cover; and
an additional drug solution discharge line connected, at one end, to the connection section of the at least one exposure prevention cover,
wherein one of the additional drug solution discharge lines is connected to the drug solution discharge line as an additional discharge line.

22. The drug solution supply system of claim 21, wherein the additional module has a plurality of exposure prevention covers, and
the plurality of additional drug solution discharge lines are serially connected to each other with the additional discharge line being at the head.

23. A drug solution supply method comprising:
a first preparation step of preparing at least one drug solution container containing a drug solution and having a connection port via which the drug solution can be discharged;
a second preparation step of housing and sealing the drug solution container in an exposure prevention cover having a connection section via which the drug solution can be discharged;
a third preparation step of connecting a drug solution line to the connection section; and
a discharge step of connecting the connection section to the connection port of the drug solution container to discharge the drug solution in the drug solution container into the drug solution line via the connection section.

24. The drug solution supply method of claim 23 further comprising:
a fourth preparation step of preparing an infusion container containing an infusion solution;
a fifth preparation step of connecting the infusion container to an infusion line that discharges the infusion solution from the infusion container; and
a step of forming a supply line capable of discharging at least one of the infusion solution and the drug solution by connecting the drug solution line and the infusion line in a trifurcated fashion,
wherein the method further comprises before the discharge step:
a first priming step of discharging the infusion solution from the infusion container toward the supply line; and
a second priming step of allowing the infusion solution to flow from the infusion container toward the connection section of the exposure prevention cover.

25. The drug solution supply method of claim 21 or 22, further comprising, after the discharge step, a flushing step of supplying an infusion solution at a position of the drug solution line near the connection section to allow the infusion solution to flow into the drug solution line.

26. The drug solution supply method of claim 23, wherein a plurality of drug solution containers are prepared in the first preparation step,
each of the plurality of drug solution containers is housed and sealed in the exposure prevention cover in the second preparation step,
the drug solution line is connected to the connection section of each of the exposure prevention covers and such drug solution lines are serially connected in the third preparation step, and
drug solutions are sequentially discharged from the drug solution containers in the discharge step.

27. The drug solution supply method of claim 26 further comprising:
a fourth preparation step of preparing an infusion container containing an infusion solution;
a fifth preparation step of connecting the infusion container to an infusion line that discharges the infusion solution from the infusion container; and
a step of forming a supply line capable of discharging at least one of the infusion solution and the drug solution by connecting the infusion line and the drug solution line placed at the head in a trifurcated fashion,
wherein a first priming step of discharging the infusion solution from the infusion container toward the supply line is executed before the step of discharging the drug solution to the head drug solution line, and
a second priming step of allowing the infusion solution to flow from the infusion container toward the connection section of the exposure prevention cover is executed before the step of discharging the drug solution to each of the drug solution lines.

28. The drug solution supply method of claim 26 or 27, further comprising a flushing step of supplying an infusion solution at a position of each of the drug solution lines near the connection section to allow the infusion solution to flow into the drug solution line, after each discharge of the drug solution from the drug solution containers in the discharge step.

29. The drug solution supply method of claim 23, wherein a plurality of drug solution containers are housed and sealed in the exposure prevention cover in the second preparation step, and
the discharge step is executed for each of the drug solution containers.

30. The drug solution supply method of claim 29 further comprising:
a fourth preparation step of preparing an infusion container containing an infusion solution;
a fifth preparation step of connecting the infusion container to an infusion line that discharges the infusion solution from the infusion container; and
a step of forming a discharge section capable of discharging at least one of the infusion solution and the drug solution by connecting the drug solution line and the infusion line in a trifurcated fashion,
wherein a first priming step of discharging the infusion solution from the infusion container toward the discharge section is executed before the step of discharging the drug solution to the head drug solution line, and
a second priming step of allowing the infusion solution to flow from the infusion container toward the connection section of the exposure prevention cover is executed before the step of discharging the drug solution from each of the drug solution containers.

31. The drug solution supply method of claim 29 or 30, further comprising a flushing step of supplying an infusion solution at a position of each of the drug solution lines near the connection section to allow the infusion solution to flow into the drug solution line, after each discharge of the drug solution from the drug solution containers in the discharge step.

32. The drug solution supply method of claim 21 further comprising:
a fourth preparation step of preparing an infusion container containing an infusion solution;
a fifth preparation step of connecting the infusion container to an infusion line that discharges the infusion solution from the infusion container;
a step of forming a supply line capable of discharging at least one of the infusion solution and the drug solution by connecting the drug solution line and the infusion line in a trifurcated fashion; and
a step of attaching, to the drug solution line, a filter that allows passage of the drug solution flowing from the drug solution container but does not allow passage of air,
wherein the method further comprises before the discharge step:
a first priming step of discharging the infusion solution from the infusion container toward the supply line.
